# EUROPEAN PATENT APPLICATION

(11) **EP 4 382 126 A1**
(43) Date of publication of application: **12.06.2024**
(21) Application number: 22852200.9
(22) Date of filing: 02.08.2022
(51) Int. Cl.: A61K 45/06, A61K 31/496, A61K 31/407, A61K 31/502, A61P 35/00, A61P 35/02

(54) **PHARMACEUTICAL COMBINATION AND USE THEREOF**

(30) Priority: 02.08.2021 WO PCT/CN2021/110099
(71) Applicant: Ascentage Pharma (Suzhou) Co., Ltd., Suzhou, Jiangsu 215000 (CN); Ascentage Pharma Group Corp Limited, Hong Kong 999077 (CN)
(72) Inventor: ZHAI, Yifan, Suzhou, Jiangsu 215000 (CN); YANG, Dajun, Suzhou, Jiangsu 215000 (CN); DENG, Jing, Suzhou, Jiangsu 215000 (CN); FANG, Douglas Dong, Suzhou, Jiangsu 215000 (CN); CARTER, Bing Z., Houston, TX 77030 (US); ANDREEFF, Michael, Houston, TX 77030 (US)
(74) Representative: AWA Denmark A/S
(86) International application number: PCT/CN2022/109762
(87) International publication number: WO 2023/011488

(57) **Abstract**

The present invention pertains to the pharmaceutical field, and particularly relates to a pharmaceutical combination comprising a Bcl-2 inhibitor or a Bcl-2/Bcl-xL inhibitor and one or more anticancer reagents, and the use of the combination to treat a disease such as cancer, espefically acute myeloid leukemia (AML), acute lymphoblastic leukemia (ALL) or multiple myeloma (MM) resistant or insensitive to Bcl-2 inhibitors and/or carrying Bcl-2 mutation and/or TP53 mutation. The invention also relates to a pharmaceutical composition or kit comprising the combination.

## Description

### Technical Field

The present invention pertains to the pharmaceutical field, and particularly relates to a pharmaceutical combination comprising a Bcl-2 inhibitor or a Bcl-2/Bcl-xL inhibitor and one or more anticancer reagents, and the use of the combination to treat a disease such as cancer. The invention also relates to a pharmaceutical composition or kit comprising the combination.

### Background of the Invention

Proliferative diseases represent a serious threat to modern society. Cancerous growths pose serious challenges for modern medicine due to their unique characteristics, including uncontrollable cell proliferation, an ability to invade local and even remote tissues, lack of differentiation, lack of detectable symptoms and lack of effective therapy and prevention. Worldwide, more than 10 million people are diagnosed with cancer every year, and cancer causes six million deaths every year or 12% of the deaths worldwide.

Many drugs targeting different targets for the treatment of cancer have been developed, including targeted-drugs (e.g., drugs targeting Bcl-2, Bcl-xL, EED, ALK, CDK4/6, Mek, MDM2, HDAC, PD-1, PARP, VEGF, BCR-ABL, IAP inhibitor etc.).

Taking Bcl-2 inhibitor as an example, apoptosis is a default mechanism by which potentially dangerous cells, e.g., cells carrying cancerous defects, are removed, and Bcl-2 family of proteins are key regulators of the mitochondrial (also called "intrinsic") pathway of apoptosis. In view of the important role for Bcl-2 family of proteins in regulating apoptosis in both cancerous and normal, i.e., non-cancerous, cells, and the recognized inter-cell type variability of Bcl-2 family protein expression, it is advantageous to have a small molecule inhibitor that selectively targets and preferably binds to one type or a subset of anti-apoptotic Bcl-2 protein(s), for example, to an anti-apoptotic Bcl-2 family member that overexpressed in a certain cancer type. Some of the Bcl-2 small molecule inhibitors have been investigated at various stages of drug development: the Bcl-2/Bcl-xL inhibitor ABT-263 (navitoclax, WO2009155386) has shown promising clinical activity in lymphoid malignancies such as chronic lymphocytic leukemia. The new generation of the BCL-2 selective inhibitor venetoclax (ABT-199/GDC-0199) was proceeded, which demonstrated robust activity in these cancers but also spared platelets (Journal of Hematology &Oncology 2015, 8, 129; Clinical Advances in Hematology &Oncology 2017, 15, 210). Currently, Venetoclax (formerly ABT-199) is approved by FDA for the treatment of patients who have relapsed or refractory chronic lymphocytic leukemia (CLL) with the 17p deletion.

However, there are still large uncertainties about which specific target drugs and which specific structure compounds are more effective in cancer treatment. Further, some drugs used alone tend to have problems of insufficient efficacy, excessive doses, and problems of drug-resistance. For example, recently, a novel Gly101Val (G101V) mutation in Bcl-2 was identified after the patients were treated with the Bcl-2 inhibitor venetoclax (ABT-199) for 19 to 42 months (Cancer Discov. 2019, 9, 342-353). This mutation dramatically reduced the binding affinity of Bcl-2 for Venetoclax (ABT-199) by about 180-fold in cell based assay.

As compared with drugs used alone, drug combinations have many potential advantages. In particular, drug combinations targeting multiple different targets may reduce the dosage of each drug, avoid the emergence of single-drug resistance, and carefully screened combinations may further produce synergistic effects, thus improving the effectiveness of cancer treatment.

Accordingly, overcoming the drug resistance of the targeted drugs as well as other anticancer reagents and improving the efficacy are some of the main objectives in drug research and development, and there is a continuous and urgent need for individual drugs and pharmaceutical combinations with improved therapeutic efficacy and/or reduced drug resistance in the field of cancer treatment.

### Summary of the Invention

In one aspect, the invention provides a pharmaceutical combination comprising a Bcl-2 inhibitor and one or more anticancer agents.

In another aspect, the invention provides a pharmaceutical composition comprising the pharmaceutical combination of the present invention, and optionally a pharmaceutically acceptable carrier.

In another aspect, the invention provides a method for treating or suppressing a cancer, reducing its severity, lowering its risk or inhibiting its metastasis in an individual, comprising administering to the individual a therapeutically effective amount of a Bcl-2 inhibitor, and optionally a therapeutically effective amount of one or more anticancer reagents.

In another aspect, the invention provides the use of a Bcl-2 inhibitor in combination with one or more anticancer reagents for treating or suppressing a cancer, reducing its severity, lowering its risk or inhibiting its metastasis in an individual.

In another aspect, the invention provides the use of a Bcl-2 inhibitor in combination with one or more anticancer reagents in the manufacture of a medicament for treating or suppressing a cancer, reducing its severity, lowering its risk or inhibiting its metastasis in an individual.

In another aspect, the invention provides the use of a MDM2 inhibitor alone or in combination with one or more anticancer reagents for treating or suppressing a cancer, reducing its severity, lowering its risk or inhibiting its metastasis in an individual.

In another aspect, the invention provides the use of a MDM2 inhibitor alone or in combination with one or more anticancer reagents in the manufacture of a medicament for treating or suppressing a cancer, reducing its severity, lowering its risk or inhibiting its metastasis in an individual.

In another aspect, the invention provides a pharmaceutical combination for treating or suppressing a cancer, reducing its severity, lowering its risk or inhibiting its metastasis in an individual comprising a MDM2 inhibitor and one or more anticancer reagents.

In another aspect, the invention provides the use of a Bcl-2/Bcl-xL inhibitor alone or in combination with one or more anticancer reagents for treating or suppressing a cancer, reducing its severity, lowering its risk or inhibiting its metastasis in an individual.

In another aspect, the invention provides the use of a Bcl-2/Bcl-xL inhibitor alone or in combination with one or more anticancer reagents in the manufacture of a medicament for treating or suppressing a cancer, reducing its severity, lowering its risk or inhibiting its metastasis in an individual.

In another aspect, the invention provides a pharmaceutical combination for treating or suppressing a cancer, reducing its severity, lowering its risk or inhibiting its metastasis in an individual comprising a Bcl-2/Bcl-xL inhibitor and one or more anticancer reagents.

In another aspect, the invention provides a kit, comprising:
(a) a first component in a first container, the first component comprising a Bcl-2 inhibitor or a Bcl-2/Bcl-xL inhibitor;
(b) a second component in a second container, the second component comprising one or more anticancer reagents, and optionally a pharmaceutically acceptable carrier; and
(c) an optional specification.

### Brief Description of the Drawings

**Figures 1A and 1B** show that Compound B can resume killing in RS4;11 cells carrying different Bcl-2 mutation.
**Figures 2A-2D** show that the combinations of the present invention could synergistically inhibit the proliferation of RS4;11 Bcl2-G101V-Flag cell lines.
**Figures 3A-3D** show that the combinations of the present invention could synergistically inhibit the proliferation of RS4;11 Bcl2-V156D-Flag cell lines.
**Figures 4A-4C** show that co-targeting BCL-2, MDM2 and IAP is most effective in Compound A induced resistant cell lines (AMI, and ALL).
**Figure 5** shows that the combination of Compound A and Compound B overcomes venetoclax-resistance in tumor models carrying BCL-2 ^{V156D} mutations (RS4;11 Bcl2-V156D-Flag cells).
**Figure 6** shows the combination treatments in RS4;11 Bcl-2-G101V-Flag human ALL cancer xenograft model in mice.
**Figures 7A and 7B** show Compound A + Compound B + Aza vs. Compound A + Compound B + Compound C in RS4;11-VEN-R human ALL cancer xenograft model in mice.
**Figures 8A and 8B** show the combination effect of Compound A+Compound C in AML cells (72 h).
**Figures 9A-9D** show that the combination treatment with Compound A and Compound C in subcutaneous MV-4-11 AML achieves synergy.
**Figures 10A-10C** show the combination treatment with Compound A and Compound C in orthotopic MOLM-13-luc human AML model.
**Figures 11A-11C** show the combination treatment with Compound A, Compound B and/or Compound C in p53 WT systemic OCI-AML 3 AML model (intrinsic resistant to venetoclax).

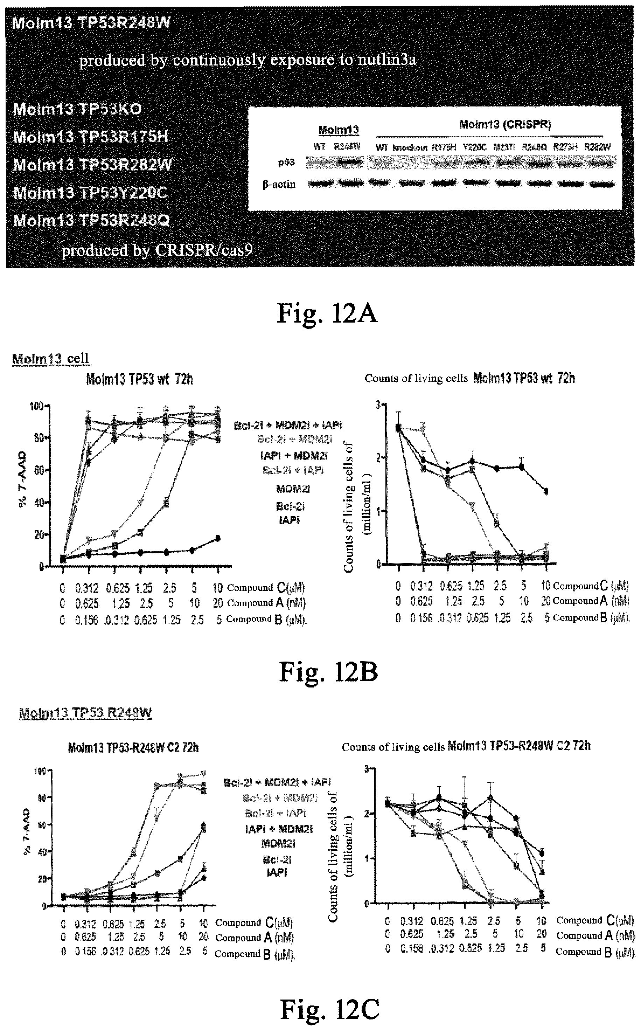

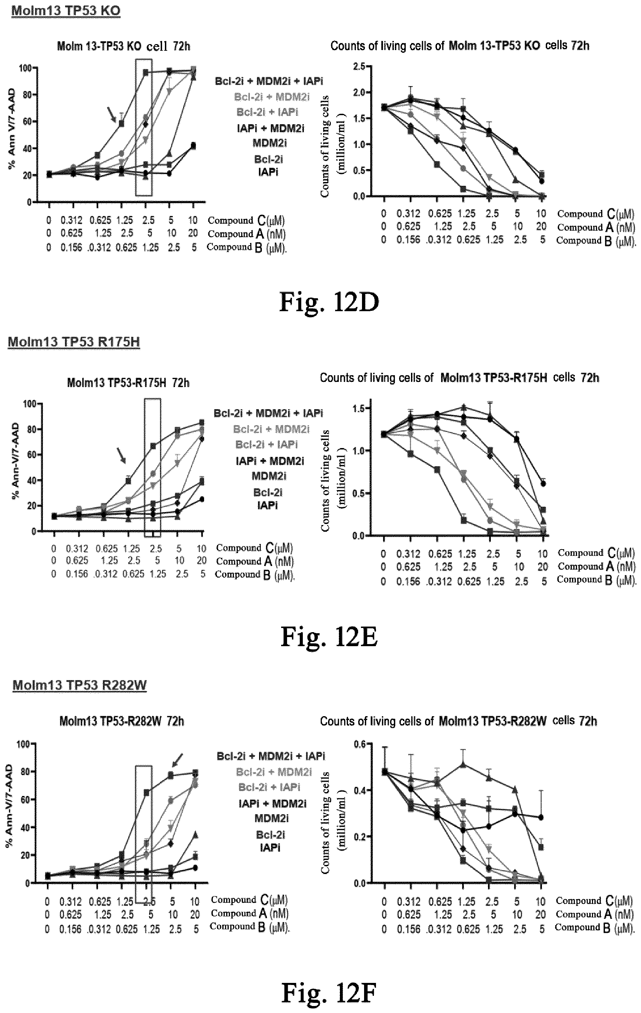

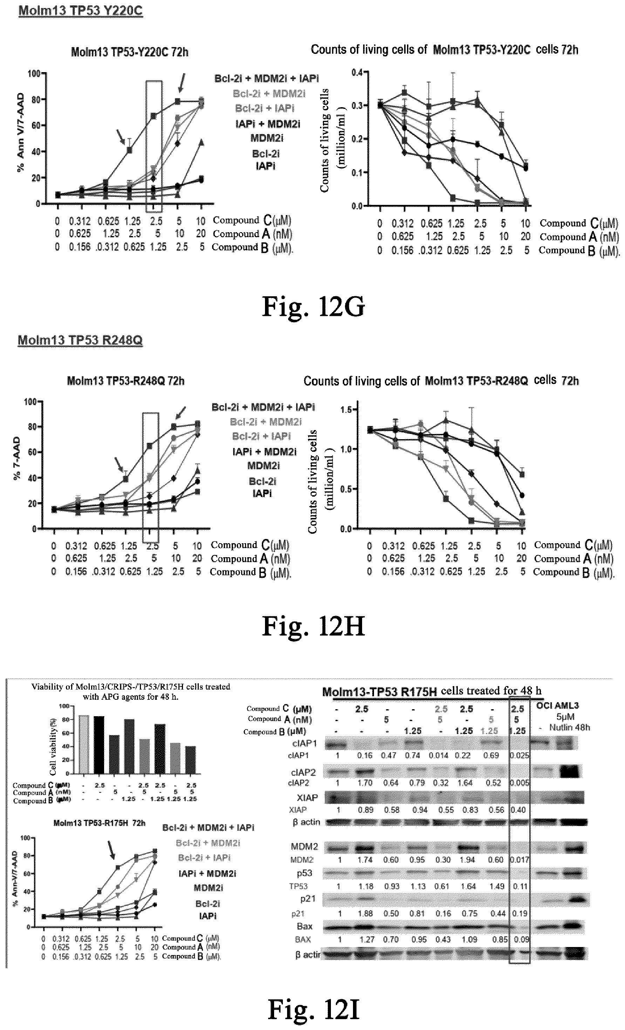

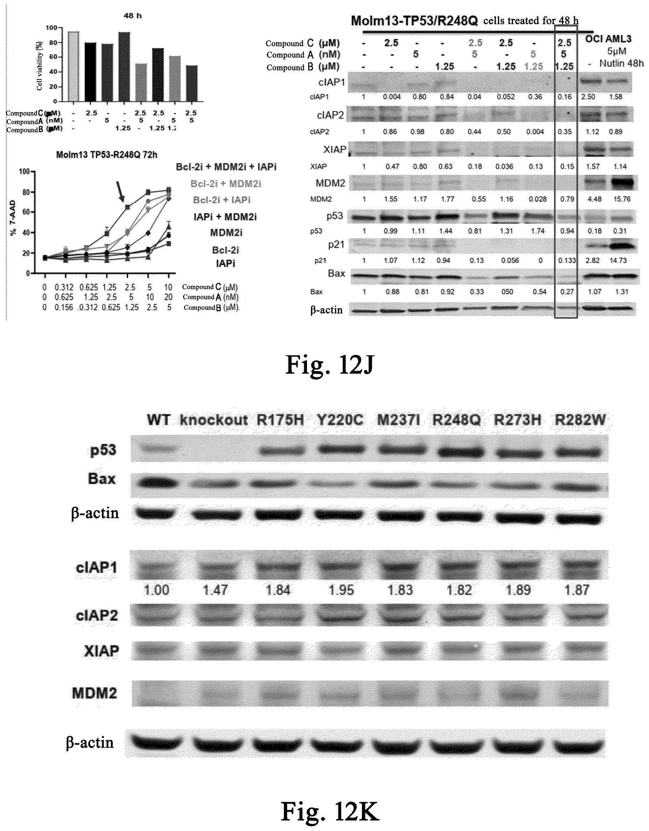

**Figures 12A-12K** show the co-targeting intrinsic and extrinsic apoptosis to maximize cell death induction in venetoclax-resistant AML cells.
**Figure 13** shows that Compound E shows single agent activity in BCL-2i insensitive cells RPMI 8226.
**Figures 14A-14D** show that Compound E and Compound C combination synergistically sensitizes BCL-2i insensitive cells to proliferation inhibition.
**Figures 15A and 15B** show that Compound C shows single agent activity in MM cells insensitive to BCL-2 inhibitor.
**Figures 16A-16F** show that compound E/compound D shows single drug activity in both Venetoclax-resistant MM cells and xenotransplantation models. Compound E inhibits the proliferation of drug-resistant clones (fig. 16A) and induces cell apoptosis (fig. 16B), and compound E is the most effective in inducing cell death (fig. 16C). Compound D, a prodrug of compound E, is also effective in the CDX models of these two cell lines (Fig. 16D and 16E), and the activity of compound E is higher than venetoclax in the MM cells of 4 MM patients (Fig. 16F).

### Detailed Description of the Invention

Unless otherwise defined below, all technical and scientific terms used herein have the same meanings as commonly understood by one of ordinary skill in the art. References to techniques used herein are intended to refer to techniques that are generally understood in the art, including those obvious changes or equivalent replacements of the techniques for those skilled in the art. While it is believed that the following terms are well understood by those skilled in the art, the following definitions are set forth to better explain the invention.

As used herein, the terms "including", "comprising", "having", "containing" or "comprising", and other variants thereof, are inclusive or open, and do not exclude other unlisted elements or method steps.

The use of the terms "a", "an", "the", and similar referents in the context of describing the invention (especially in the context of the claims) are to be construed to cover both the singular and the plural, unless otherwise indicated. Recitation of ranges of values herein merely are intended to serve as a shorthand method of referring individually to each separate value falling within the range, unless otherwise indicated herein, and each separate value is incorporated into the specification as if it were individually recited herein. The use of any and all examples, or exemplary language (e.g., "such as") provided herein, is intended to better illustrate the disclosure and is not a limitation on the scope of the disclosure unless otherwise claimed. No language in the specification should be construed as indicating any non-claimed element as essential to the practice of the disclosure.

As used herein, the terms "treat," "treating," "treatment," and the like refer to eliminating, reducing, or ameliorating a disease or condition, and/or symptoms associated therewith. Although not precluded, treating a disease or condition does not require that the disease, condition, or symptoms associated therewith be completely eliminated. The term "treat" and synonyms contemplate administering a therapeutically effective amount of a Compound of the Disclosure to a subject in need of such treatment. The treatment can be orientated symptomatically, for example, to suppress symptoms. It can be effected over a short period, be oriented over a medium term, or can be a long-term treatment, for example within the context of a maintenance therapy.

As used herein, the terms "prevent," "preventing," and "prevention" refer to a method of preventing the onset of a disease or condition and/or its attendant symptoms or barring a subject from acquiring a disease. As used herein, "prevent," "preventing," and "prevention" also include delaying the onset of a disease and/or its attendant symptoms and reducing a subject's risk of acquiring a disease. The terms "prevent," "preventing" and "prevention" may include "prophylactic treatment," which refers to reducing the probability of redeveloping a disease or condition, or of a recurrence of a previously-controlled disease or condition, in a subject who does not have, but is at risk of or is susceptible to, redeveloping a disease or condition or a recurrence of the disease or condition.

The term "synergistic effect" as used herein refers to action of two therapeutic agents, for example, slowing the symptomatic progression of a proliferative disease, particularly cancer, or symptoms thereof, which is greater than the simple addition of the effects of each drug administered by themselves. A synergistic effect can be calculated, for example, using various methods and equations well known in the art, such as those listed in the Examples of the present invention.

The term "halo" as used herein by itself or as part of another group refers to -Cl, -F, -Br, or -I.

The term "nitro" as used herein by itself or as part of another group refers to -NO₂.

The term "cyano" as used herein by itself or as part of another group refers to -CN.

The term "hydroxy" as used herein by itself or as part of another group refers to -OH.

The term "alkyl" as used herein, alone or as part of another group, refers to an unsubstituted straight or branched aliphatic hydrocarbon containing from 1 to 12 carbon atoms (ie, C₁₋₁₂ alkyl) or an indicated number of carbon atoms, for example, C₁ alkyl such as methyl, C₂ alkyl such as ethyl, C₃ alkyl such as n-propyl or isopropyl, C₁₋₃ alkyl such as methyl, ethyl, n-propyl or isopropyl, or the like. In one embodiment, the alkyl is C₁₋₄ alkyl. Non-limiting examples of C₁₋₁₂ alkyl include methyl, ethyl, n-propyl, isopropyl, n-butyl, sec-butyl, tert-butyl, isobutyl, 3-pentyl, hexyl, heptyl, octyl, nonyl and decyl. Examples of C₁₋₄ alkyl include methyl, ethyl, n-propyl, isopropyl, n-butyl, sec-butyl, tert-butyl, and isobutyl.

The term "alkenyl" as used herein by itself or as part of another group refers to an alkyl group containing one, two, or three carbon-to-carbon double bonds. In one embodiment, the alkenyl group is a C₂-C₆ alkenyl group. In another embodiment, the alkenyl group is a C₂-C₄ alkenyl group. In another embodiment, the alkenyl group has one carbon-to-carbon double bond. Non-limiting exemplary alkenyl groups include ethenyl, propenyl, isopropenyl, butenyl, sec-butenyl, pentenyl, and hexenyl.

The term "alkynyl" as used herein by itself or as part of another group refers to an alkyl group containing one, two, or three carbon-to-carbon triple bonds. In one embodiment, the alkynyl is a C₂-C₆ alkynyl. In another embodiment, the alkynyl is a C₂-C₄ alkynyl. In another embodiment, the alkynyl has one carbon-to-carbon triple bond. Non-limiting exemplary alkynyl groups include ethynyl, propynyl, butynyl, 2-butynyl, pentynyl, and hexynyl groups.

The term "haloalkyl" as used herein by itself or as part of another group refers to an alkyl group substituted by one or more fluorine, chlorine, bromine, and/or iodine atoms. In one embodiment, the alkyl is substituted by one, two, or three fluorine and/or chlorine atoms. In another embodiment, the alkyl is substituted by one, two, or three fluorine atoms. In another embodiment, the alkyl is a C₁-C₆ alkyl. In another embodiment, the alkyl is a C₁-C₄ alkyl. In another embodiment, the alkyl group is a C₁ or C₂ alkyl. Non-limiting exemplary haloalkyl groups include fluoromethyl, difluoromethyl, trifluoromethyl, pentafluoroethyl, 1,1-difluoroethyl, 2,2-difluoroethyl, 2,2,2-trifluoroethyl, 3,3,3-trifluoropropyl, 4,4,4-trifluorobutyl, and trichloromethyl groups.

The term "alkoxy" as used herein by itself or as part of another group refers to an alkyl group attached to a terminal oxygen atom. In one embodiment, the alkyl is a C₁-C₆ alkyl and resulting alkoxy is thus referred to as a "C₁-C₆ alkoxy." In another embodiment, the alkyl is a C₁-C₄ alkyl group. Non-limiting exemplary alkoxy groups include methoxy, ethoxy, and tert-butoxy.

The term "cycloalkyl" as used herein, alone or as part of another group, refers to a saturated or partially unsaturated (containing one or two double bonds) cyclic aliphatic hydrocarbon, which comprises 1 or 2 rings having 3 to 12 carbon atoms or an indicated number of carbon atoms (i.e., C₃₋₁₂ cycloalkyl). In one embodiment, the cycloalkyl has two rings. In one embodiment, the cycloalkyl has one ring. In another embodiment, the cycloalkyl group is selected from the group consisting of C₃₋₈ cycloalkyl groups. In another embodiment, the cycloalkyl group is selected from the group consisting of C₃₋₆ cycloalkyl groups. Non-limiting examples of cycloalkyl include cyclopropyl, cyclobutyl, cyclopentyl, cyclohexyl, cycloheptyl, cyclooctyl, norbornyl, decahydronaphthyl, adamantyl, cyclohexenyl, and cyclopentenyl.

The term "heterocycle" or "heterocyclyl" as used herein, alone or as part of another group, refers to a saturated or partially unsaturated (e.g., comprising one or two double bonds) cyclic group, which comprises 1, 2 or 3 rings having 3 to 14 ring members (i.e., 3- to 14-membered heterocyclyl), wherein at least one carbon atom of one of the rings is replaced by a heteroatom. Each heteroatom is independently selected from the group consisting of atoms of oxygen, sulfur (including sulfoxide and sulfone) and/or nitrogen (which may be oxidized or quaternized). The term "heterocyclyl" is intended to include a group wherein -CH₂- in the ring is replaced by -C(=O)-, for example, cyclic ureido (such as 2-imidazolidinone) and cyclic amido (such as β-lactam, γ-lactam, δ-lactam, ε-lactam) and piperazin-2-one. In one embodiment, the heterocyclyl is a 3- to 8-membered cyclic group comprising 1 ring and 1 or 2 oxygen and/or nitrogen atoms. In one embodiment, the heterocyclyl is a 4-, 5- or 6-membered cyclic group comprising 1 ring and 1 or 2 oxygen and/or nitrogen atoms. In one embodiment, the heterocyclyl is a 4- or 6-membered cyclic group comprising 1 ring and 1 or 2 oxygen and/or nitrogen atoms. The heterocyclyl can be attached to the remainder of molecule via any available carbon or nitrogen atom. Non-limiting examples of the heterocyclyl include dioxanyl, tetrahydropyranyl, 2-oxopyrrolidin-3-yl, piperazin-2-one, piperazin-2,6-dione, 2-imidazolidinone, piperidinyl, morpholinyl, piperazinyl, pyrrolidinyl and dihydroindolyl.

The term "aryl" as used herein by itself or as part of another group refers to an aromatic ring system having six to fourteen carbon atoms, i.e., C₆-C₁₄ aryl. Non-limiting exemplary aryl groups include phenyl (abbreviated as "Ph"), naphthyl, phenanthryl, anthracyl, indenyl, azulenyl, biphenyl, biphenylenyl, and fluorenyl groups. In one embodiment, the aryl group is phenyl or naphthyl. In another embodiment, the aryl group is phenyl.

The term "heteroaryl" as used herein by itself or as part of another group refers to monocyclic and bicyclic aromatic ring systems having five to 14 fourteen ring members, i.e., a 5- to 14-membered heteroaryl, comprising one, two, three, or four heteroatoms. Each heteroatom is independently oxygen, sulfur, or nitrogen. In one embodiment, the heteroaryl has three heteroatoms. In another embodiment, the heteroaryl has two heteroatoms. In another embodiment, the heteroaryl has one heteroatom. In another embodiment, the heteroaryl is a 5- to 10-membered heteroaryl. In another embodiment, the heteroaryl has 5 ring atoms, e.g., thienyl, a 5-membered heteroaryl having four carbon atoms and one sulfur atom. In another embodiment, the heteroaryl has 6 ring atoms, e.g., pyridyl, a 6-membered heteroaryl having five carbon atoms and one nitrogen atom.

The term "cancer" as used herein refers to a neoplasm or tumor caused by abnormal, uncontrolled cell growth. Non-limiting examples include those exemplary cancers described in the detailed description of the invention. The term "cancer" includes diseases involving both pre-malignant cancer cells and malignant cancer cells.

As used herein, "cancer metastasis" refers to a cancer that spreads (metastasizes) from its original site to another area of the body. Almost all cancers have the potential to metastasize. Whether metastasis will occur depends on complex interactions between multiple tumor cell factors (including type of cancer, degree of maturation (differentiation) of tumor cells, location and age of cancer, and other factors that are not fully understood). There are three ways of metastasis: local expansion from a tumor to a surrounding tissue, arrival through bloodstream to a distant site, or arrival through lymphatic system to an adjacent or distant lymph node. Each cancer can have a representative diffusion route. Tumors are named according to their primary sites (for example, breast cancer that has metastasized to the brain is called metastatic breast cancer that metastasizes to the brain).

The term "individual," "patient," or "subject" as used herein refers to including humans (e.g, patients) and animals (e.g, mice, rats, dogs, cats, rabbits, chickens, monkeys, etc.). When the subject is a human patient (usually calculated as body weight of 60 kg), a dose described herein can be obtained by conversion performed with a conversion factor for an experimental animal (e.g, human dose = mouse dose / 12.3) unless otherwise stated (Kin Tam. "Estimating the "First in human" dose-a revisit with particular emphasis on oncology drugs, ADMET & DMPK 1 (4) (2013) 63-75). Those of ordinary skill in the art can reasonably adjust the dose based on common sense and according to the specific weight of subject, the type and severity of disease, and other factors, and all of these adjusted technical solutions fall within the scope of the technical solutions claimed in the present invention.

The term "effective amount" or "prophylactically and/or therapeutically effective amount" as used herein refers to a sufficient amount (e.g, a dose) of a medicament or compound to be administered that will alleviate one or more symptoms of a disease or condition to be treated to some extent. The result can be a reduction and/or alleviation in the cause of the condition or the cause of disease or any other desired changes in biological system. For example, an "effective amount" for therapeutic use is an amount of a compound or medicament (e.g, a combination product as claimed herein) that provides a significant reduction in the clinical symptoms of the disease or condition without causing excessive toxic side effects.

The term "dose" as used herein refers to a weight (e.g, milligrams (mg)) of an active substance per kilogram (kg) of a subject's body weight.

The term "IC₅₀" as used herein refers to an amount, concentration or dose of a particularly tested compound or medicament that achieves a 50% inhibition of maximum effect in an assay that measures such effect.

The term "room temperature" as used herein refers to 25°C ± 1°C. At the same time, if the experimental temperature is not specified, it is room temperature.

The term "pharmaceutically acceptable salt", as used herein, includes both acid addition salts and base addition salts of a compound.

Suitable acid addition salts are formed from acids which form non-toxic salts. Examples include acetate, adipate, aspartate, benzoate, besylate, bicarbonate/carbonate, bisulfate/sulfate, borate, camphorsulfonate, citrate, cyclohexylaminosulfonate, ethanedisulfonate, ethanesulfonate, formate, fumarate, glucoheptonate, gluconate, glucuronate, hexafluorophosphate, hibenzate, hydrochloride/chloride, hydrobromide/bromide, hydroiodide/iodide, isethionate, lactate, malate, maleate, malonate, methanesulfonate, methyl sulfate, naphthylate, 2-naphthalenesulfonate, nicotinate, nitrate, orotate, oxalate, palmitate, pamoate, phosphate/hydrogen phosphate/dihydrogen phosphate, pyroglutamate, aldarate, stearate, succinate, tannate, tartrate, tosylate, trifluoroacetate and xinofoate.

Suitable base addition salts are formed from bases which form non-toxic salts. Examples include aluminum salts, arginine salts, benzathine benzylpenicillin salts, calcium salts, choline salts, diethylamine salts, diethanolamine salts, glycine salts, lysine salts, magnesium salts, meglumine salts, ethanolamine salts, potassium salts, sodium salts, tromethamine salts and zinc salts.

For a review of suitable salts, see "Handbook of Pharmaceutical Salts: Properties, Selection, and Use" by Stahl and Wermuth (Wiley-VCH, 2002). Methods for preparing the pharmaceutically acceptable salts of the compounds of the invention are known to those skilled in the art.

The term "solvate" as used herein is a substance formed by combination, physical binding and/or solvation of a compound of the invention with a solvent molecule, such as a disolvate, a monosolvate or a hemisolvate, wherein the ratio of the solvent molecule to the compound of the invention is about 2:1, about 1:1 or about 1:2, respectively. This kind of physical bonding involves ionization and covalent bonding (including hydrogen bonding) in different degrees. In some cases (e.g., when one or more solvent molecules are incorporated into crystal lattice of crystalline solid), the solvate can be isolated. Thus, the solvate comprises both solution phase and isolatable solvates. The compounds of the invention may be in solvated forms with pharmaceutically acceptable solvents (such as water, methanol and ethanol), and the present application is intended to encompass both solvated and unsolvated forms of the compounds of the invention.

One type of solvate is a hydrate. "Hydrate" relates to a specific subset of solvates wherein the solvent molecule is water. Solvates generally function in the form of pharmacological equivalents. The preparation of solvates is known in the art, see for example, M. Caira et al, J. Pharmaceut. Sci., 93(3): 601-611 (2004), which describes the preparation of a solvate of fluconazole with ethyl acetate and water. Similar methods for the preparation of solvates, hemisolvates, hydrates and the like are described by van Tonder et al, AAPS Pharm. Sci. Tech., 5(1): Article 12 (2004) and A.L. Bingham et al, Chem. Commun. 603-604 (2001). A representative and non-limiting method for the preparation of solvate involves dissolving a compound of the invention in a desired solvent (organic solvent, water or a mixture thereof) at a temperature above 20 °C to about 25 °C, and then the solution is cooled at a rate sufficient to form a crystal, and the crystal is separated by a known method such as filtration. Analytical techniques such as infrared spectroscopy can be used to confirm the presence of the solvent in the crystal of the solvate.

"Pharmaceutically acceptable carrier" in the context of the present invention refers to a diluent, adjuvant, excipient or vehicle together with which the therapeutic agent is administered, and which is suitable for contacting a tissue of human and/or other animals within the scope of reasonable medical judgment, and without excessive toxicity, irritation, allergic reactions, or other problems or complications corresponding to a reasonable benefit/risk ratio.

The pharmaceutically acceptable carriers that can be used in the pharmaceutical compositions or kits of the invention include, but are not limited to, sterile liquids such as water and oils, including those oils derived from petroleum, animals, vegetables or synthetic origins, for example, peanut oil, soybean oil, mineral oil, sesame oil, etc. Water is an exemplary carrier when the pharmaceutical composition is administered intravenously. It is also possible to use physiological saline and an aqueous solution of glucose and glycerin as a liquid carrier, particularly for injection. Suitable pharmaceutical excipients include starch, glucose, lactose, sucrose, gelatin, maltose, chalk, silica gel, sodium stearate, glyceryl monostearate, talc, sodium chloride, skimmed milk powder, glycerin, propylene glycol, water, ethanol and the like. The pharmaceutical composition may further contain a small amount of a wetting agent, an emulsifier or a pH buffering agent as needed. Oral formulations may contain standard carriers such as pharmaceutical grades of mannitol, lactose, starch, magnesium stearate, sodium saccharin, cellulose, magnesium carbonate, and the like. Examples of suitable pharmaceutically acceptable carriers are as described in Remington's Pharmaceutical Sciences (1990).

The pharmaceutical compositions and the components of the kit of the invention may act systemically and/or locally. For this purpose, they may be administered via a suitable route, for example by injection (e.g., intravenous, intraarterial, subcutaneous, intraperitoneal, intramuscular administration, including instillation) or transdermal administration; or by oral, buccal, nasal, transmucosal, topical administration, in form of ophthalmic preparation or by inhalation.

For these routes of administration, the pharmaceutical compositions and the components of the kit of the invention may be administered in a suitable dosage form.

The dosage forms include, but are not limited to, tablets, capsules, troches, hard candy, pulvis, sprays, creams, ointments, suppositories, gels, pastes, lotions, ointments, aqueous suspensions, injectable solutions, elixirs, syrups.

The term "container" as used herein refers to a container for holding a pharmaceutical component. This container can be used for preparation, storage, transportation and/or stand-alone/bulk sale, which is intended to include bottles, cans, vials, flasks, syringes, tubes (e.g., those used in cream products), or any other containers for preparation, containment, storage or distribution of a drug product.

The term "specification/instruction" as used herein refers to an insert, a tag, a label, etc., which records information about a pharmaceutical component located in the container. The information as recorded is typically determined by the regulatory agency (e.g., the United States Food and Drug Administration) that governs the area in which the product is to be sold. Preferably, the package leaflet specifically lists an indication for which the use of the pharmaceutical component is approved. The package leaflet can be made of any material from which information contained therein or thereon can be read. Preferably, the package leaflet is a printable material (e.g., paper, plastic, cardboard, foil, adhesive paper or plastic, etc.) on which the desired information can be formed (e.g., printed or applied).

As used herein, "resistance" refers to that a cancer cell is resistant to chemotherapy. Cancer cells may acquire resistance to chemotherapy through a range of mechanisms, including mutation or overexpression of drug targets, inactivation of drugs, or elimination of drugs from cells.

The term "about" as used herein refers to ±10%, more preferably ±5%, and most preferably ±2% of the value modified by the term, so that one of ordinary skill in the art can clearly determine the scope of the term "about" according to the modified value.

### Pharmaceutical combinations and uses

In one aspect, the invention provides a pharmaceutical combination comprising a Bcl-2 inhibitor and one or more anticancer reagents.

In a preferred embodiment, the one or more anticancer reagents are selected from the group consisting of a MDM2 inhibitor, an IAP inhibitor, and other anticancer reagents.

In a preferred embodiment, the one or more anticancer reagents are selected from the group consisting of a MDM2 inhibitor, an IAP inhibitor, and combination thereof.

In one embodiment, the Bcl-2 inhibitor is a compound of Formula V: 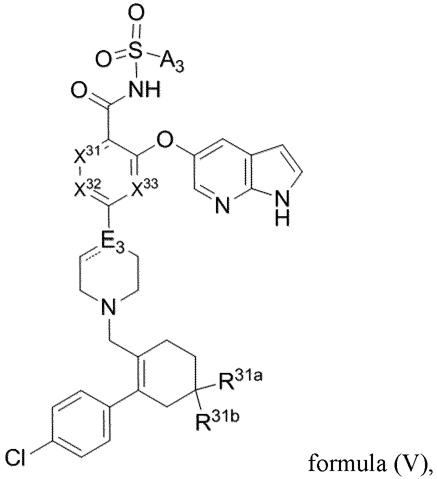 or a pharmaceutically acceptable salt or solvate thereof, wherein: 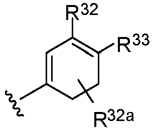
A₃ is selected from A-1 ;
E₃ is a nitrogen atom and -̅ -̅ -̅ is a single bond;
X³¹, X³², and X³³ are each independently selected from the group consisting of -CR³⁸ = and -N=;
R^{31a} and R^{31b} taken together with the carbon atom to which they are attached form a 3-, 4-, or 5-membered cycloalkyl;
R³² is selected from the group consisting of -NO₂, -SO₂CH₃, and -SO₂CF₃;
R^{32a} is selected from the group consisting of hydrogen and halogen;
R³³ is selected from -N(R^{34a})(R^{34b});
R^{34a} is selected from the group consisting of optionally substituted C₁₋₆ alkyl, optionally substituted C₃₋₆ cycloalkyl, heterocyclo, heteroalkyl, (cycloalkyl)alkyl, and (heterocyclo)alkyl;
R^{34b} is selected from the group consisting of hydrogen and C₁₋₄ alkyl;
R³⁸ is selected from the group consisting of hydrogen and halogen.

In a preferred embodiment, the Bcl-2 inhibitor is selected from the group consisting of: 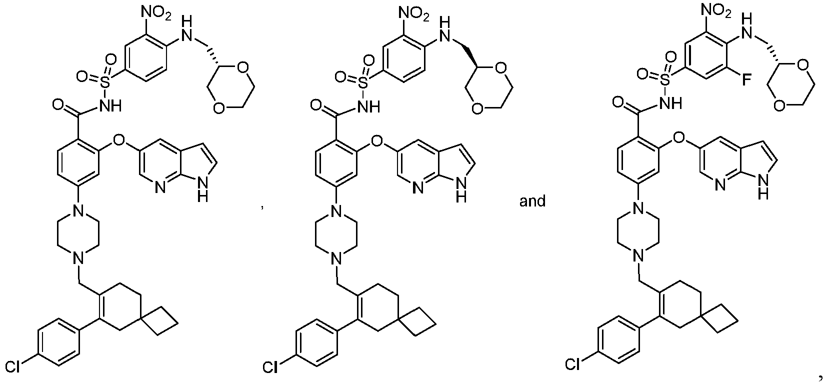 or a pharmaceutically acceptable salt or solvate thereof.

In a preferred embodiment, the Bcl-2 inhibitor is: 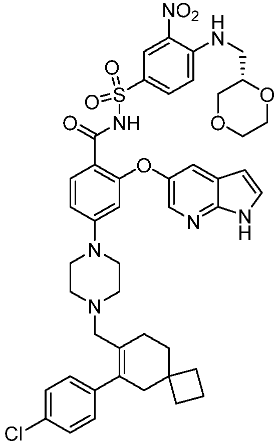 (S)-N-((4-(((1,4-dioxan-2-yl)methyl)amino)-3-nitrophenyl)sulfonyl)-2-((1H-pyrrolo[2,3-b]pyridin-5-yl)oxy)-4-(4-((6-(4-chlorophenyl)spiro[3.5]non-6-en-7-yl)methyl)piperazin-1-yl)benzamide (Compound A), or a pharmaceutically acceptable salt or solvate thereof.

In one embodiment, the MDM2 inhibitor is a compound of formula (VI), or a pharmaceutically acceptable salt or solvate thereof: 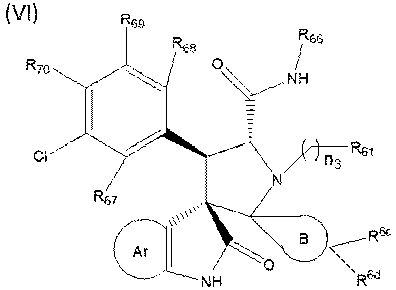 wherein 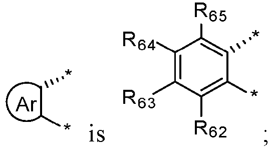
B is 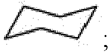 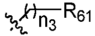
is H, CH₃, or CH₂CH₃
R₆₂, R₆₃, R₆₄, R₆₅, R₆₇, R₆₈, R₆₉, and R₇₀, independently, are selected from the group consisting of H, F, and Cl;
R₆₆ is 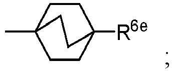 and
R^{6c} and R^{6d} are substituents on one carbon atom of ring B, wherein
R^{6c} is H, C₁₋₃ alkyl, or halo;
R^{6d} is H, C₁₋₃ alkyl, or halo;
R^{6e} is -C(=O)OR^{6a};
R^{6a} is hydrogen or unsubstituted C₁₋₄ alkyl.

In a preferred embodiment, R₆₂ is H, R₆₃ is F or Cl, and R₆₄ and R₆₅ are H, R₆₇ is fluoro, each of R₆₈, R₆₉, and R₇₀ is H, R^{6c} is H, CH₃, or halo, and R^{6d} is H, CH₃, or halo.

In a preferred embodiment, the MDM2 inhibitor is: 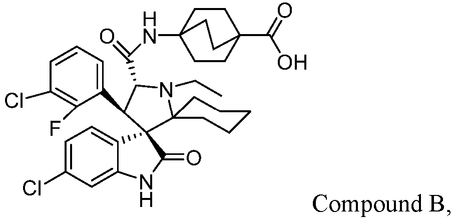 or a pharmaceutically acceptable salt or solvate thereof.

In one embodiment, the IAP inhibitor is a compound of formula (I), or a pharmaceutically acceptable salt or solvate thereof: 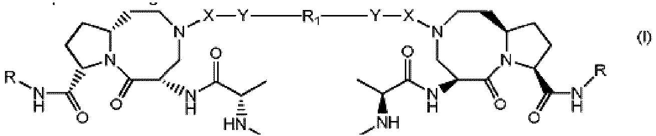 wherein
X is selected from 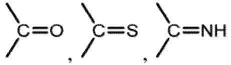 and -SO₂-;
Y is selected from -NH-, -O-, -S-, and absent;
R is selected from the group consisting of: 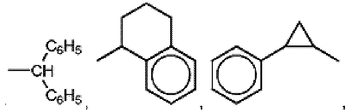 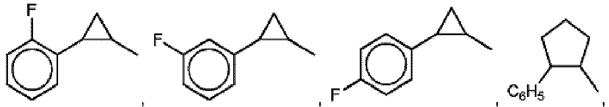 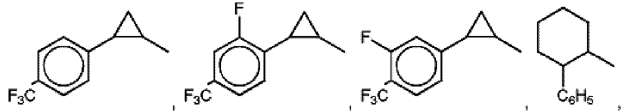 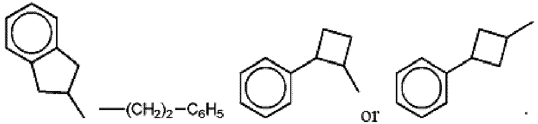
R₁ is selected from the group consisting of:

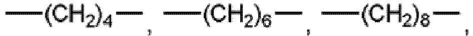

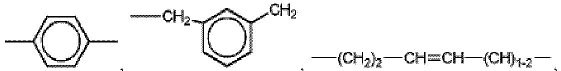

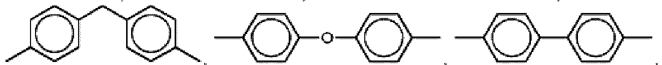

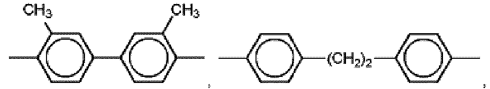

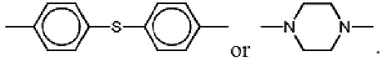

In a preferred embodiment, the MDM2 inhibitor is: 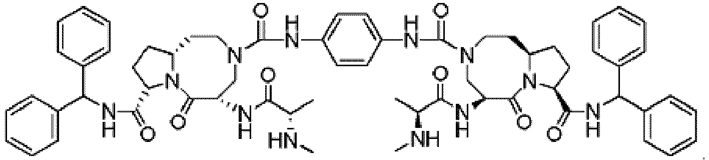 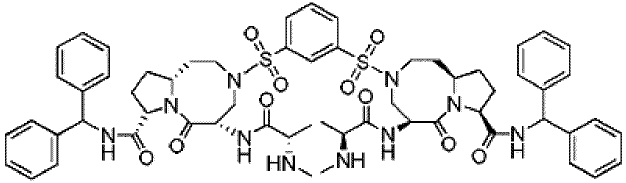 or 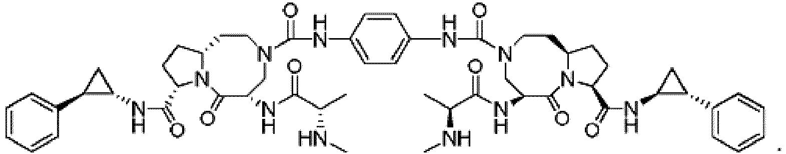 or a pharmaceutically acceptable salt or solvate thereof.

In a preferred embodiment, the MDM2 inhibitor is: 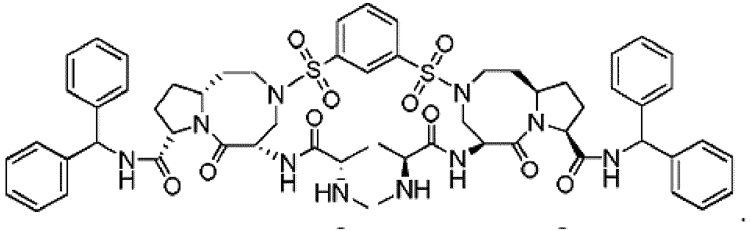 1,3-phenylenebis[7-(3S,5S,9aR)-5-((S)-2-methylamino-propionamido)-3-diphenylcarbamyl-4-oxo-3a,7-diaza-decahydrocyclopentacyclooctene)]-sulfonamide (Compound C), or a pharmaceutically acceptable salt or solvate thereof.

In a preferred embodiment, the pharmaceutical composition is for use in treating or suppressing a cancer, reducing its severity, lowering its risk or inhibiting its metastasis in an individual, and the cancer is preferably selected from the group consisting of bladder cancer, breast cancer, cervical cancer, colon cancer (including colorectal cancer), esophageal cancer, esophageal squamous cell carcinoma, head and neck cancer, liver cancer, lung cancer (including small cell lung cancer, non-small cell lung cancer and lung squamous cell carcinoma), mesothelial tumor, melanoma, myeloma, rhabdomyosarcoma, inflammatory myofibroblastic tumor, neuroturbo chargeoma, pancreatic cancer, prostate cancer, kidney cancer, renal cell carcinoma, sarcoma (including osteosarcoma), skin cancer, squamous cell carcinoma, spindle cell carcinoma, gastric cancer, testicular cancer, thyroid cancer, uterine cancer, mesothelioma, neuroblastoma, cholangiocarcinoma, leiomyosarcoma, liposarcoma, nasopharyngeal carcinoma, neuroendocrine carcinoma, ovarian cancer, salivary gland cancer, metastasis caused by spindle cell carcinoma, anaplastic large cell lymphoma, thyroid undifferentiated carcinoma, non-Hodgkin's lymphoma, Hodgkin's lymphoma, and hematological malignancies, such as acute myeloid leukemia (AML), acute lymphoblastic leukemia (ALL), diffuse large B-cell lymphoma (DLBCL), follicular lymphoma (FL), chronic lymphocytic leukemia (CLL), chronic myeloid leukemia (CML), multiple myeloma (MM), uveal melanoma, pleural mesothelioma, peritoneal mesothelioma.

In a preferred embodiment, the cancer is acute myeloid leukemia (AML), acute lymphoblastic leukemia (ALL) or multiple myeloma (MM).

In a preferred embodiment, the cancer is resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In a preferred embodiment, the cancer is carrying Bcl-2 mutation (e.g., G101V, D103E, V156D and combination thereof) and/or TP53 mutation (e.g., R248Q, R175H, R282W, Y220C and combination thereof), further preferably, the cancer is acute myeloid leukemia (AML), acute lymphoblastic leukemia (ALL) or multiple myeloma (MM) carrying Bcl-2 mutation and/or TP53 mutation.

In a preferred embodiment, the weight ratio between the Bcl-2 inhibitor and the one or more anticancer reagents is 0.005-5000 : 0.005-5000, for example, 0.05-1500 : 0.005-5000, 0.1-6 : 0.005-4, 100 : 0.5-400, 100 : 1-350, 100 : 2-300, 100 : 5-200, 100 : 10-150, 100 : 10-100, 100 : 10-90, or 100: 20-80.

In a preferred embodiment, the molar ratio between the Bcl-2 inhibitor and the one or more anticancer reagents is 10-1: 1-10, for example, 10:1, 9:1, 8:1, 7:1, 6:1, 5:1, 4:1, 3:1, 2:1, 1:1: 1:2, 1:3, 1:4, 1:5, 1:6, 1:7, 1:8, 1:9, 1:10, and the ranges between any of the aforementioned values are also included.

In a preferred embodiment, the Bcl-2 inhibitor is: 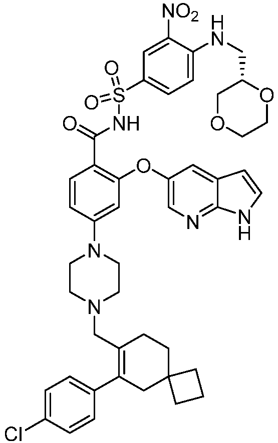
(S)-N-((4-(((1,4-dioxan-2-yl)methyl)amino)-3-nitrophenyl)sulfonyl)-2-((1H-pyrrolo[2,3-b]pyridin-5-yl)oxy)-4-(4-((6-(4-chlorophenyl)spiro[3.5]non-6-en-7-yl)methyl)piperazin-1-yl)benzamide (Compound A), or a pharmaceutically acceptable salt or solvate thereof; and/or
the MDM2 inhibitor is: 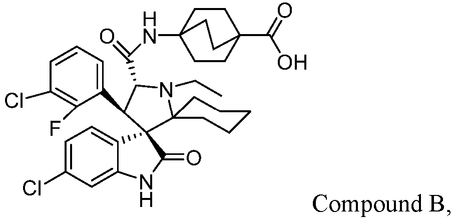
or a pharmaceutically acceptable salt or solvate thereof; and/or
the MDM2 inhibitor is:

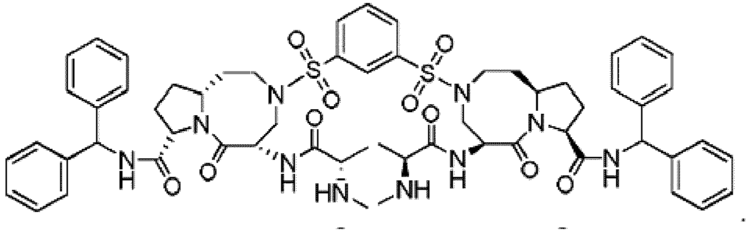

1,3-phenylenebis[7-(3S,5 S,9aR)-5-((S)-2-methylamino-propionamido)-3-diphenylcarbamyl-4-oxo-3a,7-diaza-decahydrocyclopentacyclooctene)]-sulfonamide (Compound C), or a pharmaceutically acceptable salt or solvate thereof.

In another aspect, the present invention provides a pharmaceutical composition comprising any pharmaceutical combination of the present invention, and optionally a pharmaceutically acceptable carrier.

In a preferred embodiment, the pharmaceutical composition may be in any form, for example, the composition is in the form of a tablet, a capsule, a granule, a syrup, a powder, a lozenge, a sachet, a cachet, an elixir, a suspension, an emulsion, a solution, a syrup, an aerosol, an ointment, a cream and an injection.

The pharmaceutical combinations typically are administered in admixture with a pharmaceutical carrier to give a pharmaceutical composition selected with regard to the intended route of administration and standard pharmaceutical practice. Pharmaceutical compositions for use in accordance with the present invention are formulated in a conventional manner using one or more physiologically acceptable carriers comprising excipients and/or auxiliaries that facilitate processing of the pharmaceutical combinations.

These pharmaceutical compositions can be manufactured, for example, by conventional mixing, dissolving, granulating, dragee-making, emulsifying, encapsulating, entrapping, or lyophilizing processes. Proper formulation is dependent upon the route of administration chosen. When a therapeutically effective amount of the pharmaceutical combination is administered orally, the composition typically is in the form of a tablet, capsule, powder, solution, or elixir. When administered in tablet form, the composition additionally can contain a solid carrier, such as a gelatin or an adjuvant. The tablet, capsule, and powder contain about 0.01% to about 95%, and preferably from about 1% to about 50%, of the pharmaceutical combination. When administered in liquid form, a liquid carrier, such as water, petroleum, or oils of animal or plant origin, can be added. The liquid form of the composition can further contain physiological saline solution, dextrose or other saccharide solutions, or glycols. When administered in liquid form, the composition contains about 0.1% to about 90%, and preferably about 1% to about 50%, by weight, of the pharmaceutical combination.

When a therapeutically effective amount of pharmaceutical combination is administered by intravenous, cutaneous, or subcutaneous injection, the composition is in the form of a pyrogen-free, parenterally acceptable aqueous solution. The preparation of such parenterally acceptable solutions, having due regard to pH, isotonicity, stability, and the like, is within the skill in the art. A preferred composition for intravenous, cutaneous, or subcutaneous injection typically contains, an isotonic vehicle.

The pharmaceutical combination can be readily combined with pharmaceutically acceptable carriers well-known in the art. Standard pharmaceutical carriers are described in Remington's Pharmaceutical Sciences, Mack Publishing Co., Easton, PA, 19th ed. 1995. Such carriers enable the active agents to be formulated as tablets, pills, dragees, capsules, liquids, gels, syrups, slurries, suspensions and the like, for oral ingestion by a subject to be treated. Pharmaceutical preparations for oral use can be obtained by adding the pharmaceutical combination to a solid excipient, optionally grinding the resulting mixture, and processing the mixture of granules, after adding suitable auxiliaries, if desired, to obtain tablets or dragee cores. Suitable excipients include, for example, fillers and cellulose preparations. If desired, disintegrating agents can be added.

The pharmaceutical combination can be formulated for parenteral administration by injection, e.g., by bolus injection or continuous infusion. Formulations for injection can be presented in unit dosage form, e.g., in ampules or in multidose containers, with an added preservative. The compositions can take such forms as suspensions, solutions, or emulsions in oily or aqueous vehicles, and can contain formulatory agents such as suspending, stabilizing, and/or dispersing agents.

Pharmaceutical compositions for parenteral administration include aqueous solutions of the active agent in water-soluble form. Additionally, suspensions of the pharmaceutical combination can be prepared as appropriate oily injection suspensions. Suitable lipophilic solvents or vehicles include fatty oils or synthetic fatty acid esters. Aqueous injection suspensions can contain substances which increase the viscosity of the suspension. Optionally, the suspension also can contain suitable stabilizers or agents that increase the solubility of the compounds and allow for the preparation of highly concentrated solutions. Alternatively, a present composition can be in powder form for constitution with a suitable vehicle, e.g., sterile pyrogen-free water, before use.

The pharmaceutical combination also can be formulated in rectal compositions, such as suppositories or retention enemas, e.g., containing conventional suppository bases. In addition to the formulations described previously, the Compound of the Disclosure also can be formulated as a depot preparation. Such long-acting formulations can be administered by implantation (for example, subcutaneously or intramuscularly) or by intramuscular injection. Thus, for example, the Compound of the Disclosure can be formulated with suitable polymeric or hydrophobic materials (for example, as an emulsion in an acceptable oil) or ion exchange resins.

In particular, the pharmaceutical combination can be administered orally, buccally, or sublingually in the form of tablets containing excipients, such as starch or lactose, or in capsules or ovules, either alone or in admixture with excipients, or in the form of elixirs or suspensions containing flavoring or coloring agents. Such liquid preparations can be prepared with pharmaceutically acceptable additives, such as suspending agents. The pharmaceutical combination also can be injected parenterally, for example, intravenously, intramuscularly, subcutaneously, or intracoronarily. For parenteral administration, the pharmaceutical combination are typically used in the form of a sterile aqueous solution which can contain other substances, for example, salts or monosaccharides, such as mannitol or glucose, to make the solution isotonic with blood.

In another aspect, the invention provides a method for treating or suppressing a cancer, reducing its severity, lowering its risk or inhibiting its metastasis in an individual, comprising administering to the individual a therapeutically effective amount of a Bcl-2 inhibitor, and optionally a therapeutically effective amount of one or more anticancer reagents.

In a preferred embodiment, the one or more anticancer reagents are selected from the group consisting of a MDM2 inhibitor, an IAP inhibitor, and other anticancer reagents.

In a preferred embodiment, the Bcl-2, MDM2 and/or IAP inhibitor is as defined above and the cancer is as defined above.

In a preferred embodiment, the Bcl-2 inhibitor is administrated in an amount of from about 0.005 mg/day to about 5000 mg/day, such as an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In a preferred embodiment, the Bcl-2 inhibitor is administrated in an amount of from about 1 ng/kg to about 200 mg/kg, about 1 µg/kg to about 100 mg/kg, or about 1 mg/kg to about 50 mg/kg per unit dose, for example, administrated in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg per unit dose, and administrated with one or more (e.g., 1, 2, 3, 4, 5, 6, 7, 8, 9 or 10) unit doses per day.

In a preferred embodiment, the one or more anticancer reagents are administrated in an amount of from 0.005 mg/day to about 5000 mg/day, for example, about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In a preferred embodiment, the one or more anticancer reagents are administrated in an amount of from about 1 ng/kg to about 200 mg/kg, from about 1 µg/kg to about 100 mg/kg, or from about 1 mg/kg to about 50 mg/kg per unit dose, for example, administrated in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg /kg, about 70 mg/kg, about 80 mg/kg, about 90 mg/kg, about 100 mg/kg, about 125 mg/kg, about 150 mg/kg, about 175 mg/kg, about 200 mg/kg per unit dose, and administered with one or more (e.g., 1, 2, 3, 4, 5, 6, 7, 8, 9, or 10) unit doses per day.

In a preferred embodiment, the Bcl-2 inhibitor, and the one or more anticancer reagents are administered together, simultaneously, sequentially or alternately.

In a preferred embodiment, the Bcl-2 inhibitor, and the one or more anticancer reagents are administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days.

In a preferred embodiment, the Bcl-2 inhibitor, and the one or more anticancer reagents are administered for one or more (e.g., 1, 2, 3, 4, 5, 6, 7, 8, 9 or 10) courses of treatment, in which each of the courses lasts at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days; and there is an interval of 0, 1, 2, 3, 4, 5, 6, 7, 8, 9, 10 days, two weeks, three weeks or four weeks between every two courses of treatment.

In a preferred embodiment, when there are a plurality of courses of treatment, the amount of the Bcl-2 inhibitor and/or anticancer reagents administered in each course of treatment is same or different. In a more preferred embodiment, the amount of the Bcl-2 inhibitor and/or anticancer reagents administered during the previous course of treatment is 1-10 times, preferably 1-5 times, such as 1.5, 2, 2.5, 3, 3.5, 4, 4.5 or 5 times, the amount administered during the subsequent course of treatment.

In a preferred embodiment, the Bcl-2 inhibitor, and the one or more anticancer reagents are administrated via the same (e.g., oral) or different routes (e.g., oral and parenteral (e.g., injection), respectively).

In a preferred embodiment, the anticancer reagent is administrated in a lower dose in comparison with the dose of the anticancer reagent that is administered alone or when the one or more Bcl-2 inhibitors are not administered.

In a preferred embodiment, the Bcl-2 inhibitor enhances the therapeutic efficacy of the anticancer reagent in treatment of a cancer and/or reducing a side-effect of the anticancer reagent in treatment of a cancer.

In a preferred embodiment, the one or more anticancer reagents can reduce or overcome the resistance or insensitivity of cancer to the Bcl-2 inhibitor.

In a preferred embodiment, the Bcl-2 inhibitor and one or more anticancer reagents (e.g., those selected from the group consisting of a MDM2 inhibitor, an IAP inhibitor, and other inhibitors) achieve synergistic effect (synergy) in treatment of a cancer and/or reducing a side-effect of the anticancer reagent in treatment of a cancer.

In another aspect, the invention provides the use of a Bcl-2 inhibitor in combination with one or more anticancer reagents (e.g., those selected from the group consisting of a MDM2 inhibitor, an IAP inhibitor, and other anticancer reagents) for treating or suppressing a cancer, reducing its severity, lowering its risk or inhibiting its metastasis in an individual.

In a preferred embodiment, the Bcl-2, MDM2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides the use of a Bcl-2 inhibitor in combination with one or more anticancer reagents (e.g., those selected from the group consisting of a MDM2 inhibitor, an IAP inhibitor, and other anticancer reagents) in the mamufacture of a medicament for treating or suppressing a cancer, reducing its severity, lowering its risk or inhibiting its metastasis in an individual.

In a preferred embodiment, the Bcl-2, MDM2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides the use of a MDM2 inhibitor alone or in combination with one or more anticancer reagents (e.g., those selected from the group consisting of a Bcl-2 inhibitor, an IAP inhibitor, and other anticancer reagents) for treating or suppressing a cancer, reducing its severity, lowering its risk or inhibiting its metastasis in an individual.

In a preferred embodiment, the MDM2, Bcl-2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides the use of a MDM2 inhibitor alone or in combination with one or more anticancer reagents (e.g., those selected from the group consisting of a Bcl-2 inhibitor, an IAP inhibitor, and other anticancer reagents) in the manufacture of a medicament for treating or suppressing a cancer, reducing its severity, lowering its risk or inhibiting its metastasis in an individual.

In a preferred embodiment, the MDM2, Bcl-2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides a pharmaceutical combination for treating or suppressing a cancer, reducing its severity, lowering its risk or inhibiting its metastasis in an individual comprising a MDM2 inhibitor and one or more anticancer reagents (e.g., those selected from the group consisting of a Bcl-2 inhibitor, an IAP inhibitor, and other anticancer reagents).

In a preferred embodiment, the MDM2, Bcl-2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides the use of a Bcl-2/Bcl-xL inhibitor alone or in combination with one or more anticancer reagents (e.g., those selected from the group consisting of a MDM2 inhibitor, an IAP inhibitor, and other anticancer reagents) for treating or suppressing a cancer, reducing its severity, lowering its risk or inhibiting its metastasis in an individual.

In a preferred embodiment, the MDM2 inhibitor and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In a preferred embodiment, the Bcl-2/Bcl-xL inhibitor is selected from: 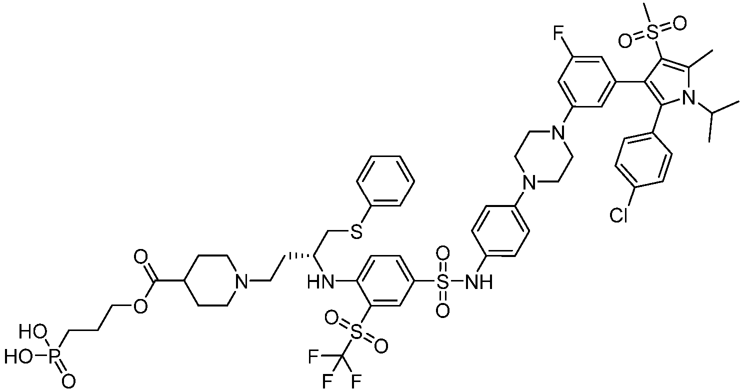 (3R)-1-(3-(4-(4-(4-(3-(2-(4-chlorophenyl)-1-isopropyl-4-methylsulfonyl-5-methyl-1H-pyrrol-3-yl)-5-fluorophenyl)piperazin-1-yl)-phenylaminosulfonyl)-2-trifluoromethylsulfonyl-anilino)-4-phenylthio-butyl)-piperidine-4-carboxylic acid 3-phosphonopropyl ester (Compound D), or a pharmaceutically acceptable salt or solvate thereof; or 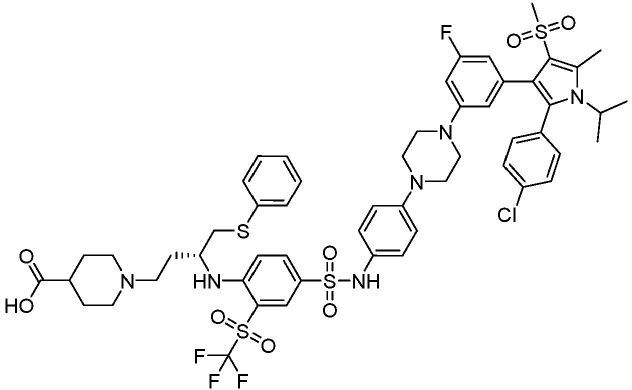 ((R)-1-(3-((4-(N-(4-(4-(3-(2-(4-chlorophenyl)-1-isopropyl-5-methyl-4-(methylsulfonyl)-1H-pyrrol-3-yl)-5-fluorophenyl)piperazin-1-yl)phenyl)sulfamoyl)-2-((trifluoromethyl)sulfonyl)phenyl)amino)-4-(phenylthio)butyl)piperidine-4-carboxylic acid (Compound E), or a pharmaceutically acceptable salt or solvate thereof.

In another aspect, the invention provides the use of a Bcl-2/Bcl-xL inhibitor alone or in combination with one or more anticancer reagents (e.g., those selected from the group consisting of a MDM2 inhibitor, an IAP inhibitor, and other anticancer reagents) in the manufacture of a medicament for treating or suppressing a cancer, reducing its severity, lowering its risk or inhibiting its metastasis in an individual.

In a preferred embodiment, the Bcl-2/Bcl-xL, MDM2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides a pharmaceutical combination for treating or suppressing a cancer, reducing its severity, lowering its risk or inhibiting its metastasis in an individual comprising a Bcl-2/Bcl-xL inhibitor and one or more anticancer reagents (e.g., those selected from the group consisting of a MDM2 inhibitor, an IAP inhibitor, and other anticancer reagents).

In a preferred embodiment, the Bcl-2/Bcl-xL, MDM2 and/or IAP inhibitor is as defined above and/or the cancer is as defined above.

In a preferred embodiment, the individual suffers from cancer resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

In another aspect, the invention provides a kit, comprising:
(a) a first component in a first container, the first component comprising a Bcl-2 inhibitor (preferably a Bcl-2 inhibitor as defined above) or a Bcl-2/Bcl-xL inhibitor (preferably a Bcl-2/Bcl-xL inhibitor as defined above), and optionally a pharmaceutically acceptable carrier;
(b) a second component in a second container, the second component comprising one or more anticancer reagents (preferably an anticancer reagent as defined above), and optionally a pharmaceutically acceptable carrier; and
(c) an optional specification,
wherein the first container and the second container may be the same or different.

### Examples

In order to make the objects and technical solutions of the present invention clearer, the present invention will be further described below in conjunction with specific example. It should be understood that the examples are not intended to limit the scope of the invention. Further, specific experimental methods not mentioned in the following examples were carried out in accordance with a conventional experimental method.

### Synthesis of compounds

### Example 1: Preparation of exemplary compounds A-E.

Compound A was prepared according to the method knowns in the art, such as those described in WO 2018/027097A1.
Compound B was prepared according to the method knowns in the art, such as those described in WO2015/161032A1.
Compound C was prepared according to the method knowns in the art, such as those described in WO 2014/031487A1.
Compounds D and E were prepared according to the method knowns in the art, such as those described in WO 2014/113413A1.

### Biological Assays

General experimental methods used in the invention are as follows:

### WST experiment

Cell plating: Anti-proliferative effects were detected by a CCK-8 (Cell Counting Kit-8) assay based on water soluble tetrazolium salt (WST). The cells were seeded in 96-well plates, and only 95 µL of complete medium was added to each negative control group. 95 µL of complete medium cell suspension was added to each well to be tested, and the cell density was (5-10)×10^4/ hole.

Dosing (protection from light): In 96-well culture plates, according to the sensitivity of different cells to different drugs, the highest concentration was selected as 10 µM, and 9 concentrations were obtained by serial dilution in a ratio of 1:3. 5 µL of compound was added to each well and 2-3 replicate wells were made for per concentration. After the compound was added, 96-well plates were incubated in a 5% CO₂ incubator at 37 °C. After 72 hours of action by using 9 different concentrations of the drug with 3 fixed doses of Compound 5, the combination effect of Compound 5 and the drug was tested.

Reading: At the end of the culture, the old solution was removed from the well to be tested, and 100 µl/well CCK-8 test solution (containing 10% CCK-8, 5% FBS in the corresponding medium) was added. The plates were continuously incubated at 37 °C for 2-4 hours in a CO₂ incubator.

The OD values were measured using a microplate reader (SpectraMax Plus 384, Molecular Devices, LLC., US) under A450 nm. Using the average OD value of 3 replicate wells, the percentage of cell viability was calculated by the following formula: (O.D. of test well - O.D. of blank control well) / (O.D. of cell control well - O.D. of blank control well) × 100%.

For combination experiments, cell viability was calculated by normalization of the mean OD values of 3 replicate wells of single drug control. The comparison of the IC50 values obtained from the curves of combined drugs of administration and single drug of administration shows that the two compounds achieved synergistic effect (the curve of the combined drugs of administration shifted to the left).

### Cell viability assays (CTG)

Cell viability was determined using CellTiter-Glo^{®} luminescent cell viability assay (Promega) or WST assay (Cell counting Kit-8, Shanghai life iLab, China) by following manufacturer's instruction. Cell viability was calculated as cell viability = (mean RLU sample - mean RLU blank) / (RLU cell control - RLU blank) × 100. IC₅₀ value was calculated using GraphPad Prism. Combination index (CI) value was calculated by CalcuSyn software (BIOSOFT, UK). CI<0.9 indicate a synergistic combination effect. CI<0.1 scored as 5+ indicates very strong synergistic combination effect, CI between 0.1 and 0.3 scored as 4+ indicates strong synergistic combination effect, CI between 0.3 and 0.7 scored as 3+ indicates medium synergistic combination effect.

### Evaluation method for in vivo pharmacodynamic experiment

A subcutaneous xenograft tumor model of human tumor immunodeficient mice was established by cell inoculation: tumor cells in logarithmic growth phase were collected, counted, resuspended in 1×PBS, and the cell suspension concentration was adjusted to 2.5-5×10⁷/mL. The tumor cells were inoculated subcutaneously in the right side of immunodeficient mice with a 1 mL syringe (4 gauge needle), 5-10×10⁶/0.2 mL/mouse. All animal experiments were strictly in accordance with the specifications for the use and management of experimental animals in GenePharma Co., Ltd. and Suzhou Ascentage Pharma Co., Ltd. The calculation of relevant parameters refers to the Chinese NMPA "Guidelines for Non-Clinical Research Techniques of Cytotoxic Antitumor Drugs".

Animal body weight and tumor size were measured twice weekly during the experiment. The state of the animal and the presence or absence of death were observed every day. The growth of tumor and the effects of treatment on normal behavior of animals were monitored routinely, specifically involving experimental animal activity, feeding and drinking, weight gain or loss, eyes, clothing hair and other abnormalities. The deaths and clinical symptoms observed during the experiment were recorded in the raw data. All operations for administration and measurement of mouse body weight and tumor volume were performed in a clean bench. According to the requirements of the experimental protocol, after the end of the last administration, plasma and tumor tissues were collected, weighed and photographed. The plasma and tumor samples were frozen at -80 °C for ready-to-use.

Tumor volume (TV) is calculated as: TV = a×b²/2, wherein a and b represent the length and width of the tumor to be measured, respectively.

The relative tumor volume (RTV) is calculated as: RTV = Vₜ/V₁, wherein V₁ is the tumor volume at the start of grouping and administration, and Vₜ is the tumor volume measured on the t day after administration.

The evaluation index of anti-tumor activity is the relative tumor proliferation rate T/C (%), and the calculation formula thereof is: relative tumor proliferation rate T/C (%) = (T_{RTV}/C_{RTV}) × 100%, T_{RTV} is the RTV of treatment group, C_{RTV} is the RTV of solvent control group.

Tumor regression rate (%) is calculated as: the number of tumor-bearing mice which exhibit SD (stable disease), PR (partial regression) and CR (complete regression) after treatment / the total number of the mice in this group × 100%. Change of body weight (%) = (measured body weight - body weight at the start of grouping) / body weight at the start of grouping × 100%.

Evaluation criteria for therapeutic efficiency: According to the Chinese NMPA "Guidelines for Non-Clinical Research Techniques of Cytotoxic Anti-tumor Drugs" (November 2006), when T/C (%) value is ≤40% and statistical analysis shows p<0.05, efficiency is confirmed. A dose of drug is considered to be severely toxic if the body weight of mouse is reduced by more than 20% or the number of drug-related deaths exceeds 20%.

According to the description by Clarke R., Issues in experimental design and endpoint analysis in the study of experimental cytotoxic agents in vivo in breast cancer and other models [J]. Breast Cancer Research & Treatment, 1997, 46(2-3): 255-278, synergy analysis was evaluated using the following formula: synergy factor = ((A/C) × (B/C)) / (AB/C); A = RTV value of drug A alone group; B = RTV value of drug B alone group; C = RTV value of the solvent control group, and AB= RTV value of the A and B combination group. Synergistic factor >1 indicates that synergy is achieved; synergy factor = 1 indicates that additive effect is achieved; and synergy factor <1 indicates that antagonistic effect is achieved.

Use of mRECIST (Gao et al., 2015) measured tumor responses included stable disease (SD), partial tumor regression (PR), and complete regression (CR), determined by comparing tumor volume change at day t to its baseline: tumor volume change (%) = (Vt-V1/V1). The BestResponse was the minimum value of tumor volume change (%) for t ≥ 10. For each time t, the average of tumor volume changes from t = 1 to t was also calculated. BestAvgResponse was defined as the minimum value of this average for t ≥ 10. The criteria for response (mRECIST) were adapted from RECIST criteria (Gao et al., 2015; Therasse et al., 2000) and defined as follows: mCR, BestResponse < -95% and BestAvg Response<-40%; mPR, BestResponse < -50% and BestAvgResponse < -20%; mSD, BestResponse < 35% and BestAvgResponse < 30%; mPD, not otherwise categorized. SD, PR, and CR were considered responders and used to calculate response rate (%). Body weight of animals were monitored simultaneously. The change in body weight was calculated based on the animal weight of the first day of dosing (day 1). Tumor volume and changes in body weight (%) were represented as the mean ± standard error of the mean (SEM).

### Orthotopic/systemic xenograft method

The MOLM 13-Luc tumor cells were maintained in vitro as suspension culture in RPMI 1640 medium supplemented with 10 % heat inactivated fetal bovine serum (Gibco product), 100 U/mL penicillin and 100 µg/mL streptomycin, at 37 °C in an atmosphere of 5 % CO2 in air. The tumor cells were routinely subcultured twice weekly. The cells growing in an exponential growth phase were harvested and counted for tumor inoculation.

In order to suppress the immune system for establishing xenograft models, the female NOD SCID mice were pre-treated with 2 times of 150 mg/kg cyclophosphamide, qd, at 24 hours before cell inoculation. Each mouse was inoculated with MOLM 13-luc tumor cells (2 × 10⁶/mouse) in 0.2 mL of PBS via tail vein for tumor development. Animals were selected for grouping 3 days after tumor implantation when the average bioluminescence measurement reached 3.18 × 10⁷ photons/sec. The animals were assigned into groups using an Excel-based randomization software performing stratified randomization based upon their bioluminescence intensity. Treatment was immediately initiated on the same day (defined as d1).

The first endpoint is to assess if the tumor growth rate can be delayed and/or tumor metastasis can be prevented in comparison with the vehicle-treated group. To this end, the surgically inoculated mice were weighed and intraperitoneally administered luciferin at a dose of 150 mg/kg. Ten minutes after the luciferin injection, the animals were anesthetized with the mixture gas of oxygen and isoflurane. When the animals were in a complete anesthetic state, the mice were moved into the imaging chamber of an IVIS (Lumina II) imaging system for bioluminescence measurements. The bioluminescence was measured and recorded twice per week. Tumor growth curve is plotted with bioluminescence intensity (photons/sec). The tumor bioluminescence was then used for the calculation of T/C value (%), T and C are the relative bioluminescence growth rate of the treated and control groups, respectively, on a given day. A one-way ANOVA was performed on the tumor bioluminescence data and a significant F - statistics (a ratio of treatment variance to the error variance) was obtained (P < 0.001), comparisons between groups were carried out with Games - Howell.

The second endpoint is animal survival. Animals were checked daily and the ones that showed deteriorating and moribund conditions (including body weight lost > 20%, limb paralysis, eye protruding, and abdominal girth enlargement due to tumor growth and metastasis) and/or lost the ability to acquire adequate food or water were euthanized with CO₂. The survival time of each animal was recorded and median survival time (MST) was calculated for each group. The survival time was analyzed by Kaplan-Meier method. The event of interest is the animal death. The survival time is defined as the time (in days) from the treatment start to death. For each group, the median survival time and corresponding 95% confidence interval were calculated. The Kaplan-Meier curves were also constructed for each group and the log-rank test was used to compare survival curves between groups. The statistically significant difference in survival time was analyzed between vehicle group and all treated groups, and among the treated groups as well.

All data were analyzed using SPSS 17.0. A P value < 0.05 was considered to be statistically significant.

### Example 2: Compound B can resume killing in RS4;11 cells carrying different Bcl-2 mutation

Method used was the cell viability CTG assay. RS4;11 cells were established from the bone marrow of a patient with acute lymphoblastic leukemia (ALL).

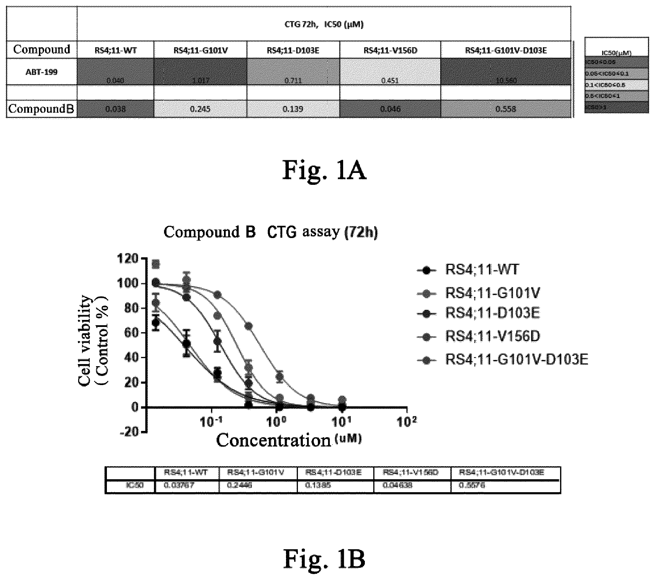

As shown in Fig. 1A and Fig. 1B, Compound B could effectively decrease cell viability of RS4;11 cells, including RS4;11-WT (wild type) and RS4;11 carrying different Bcl-2 mutation such as G101V, D103E, V156D and G101V-D103E. The IC50 (CTG 72h) values of Compound B are lower than those of ABT-199 (venetoclax) in all cell lines.

### Conclusion:

Compound B can resume killing in RS4;11 cells carrying different Bcl-2 mutation. Since BCL2 mutations are widely reported in venetoclax-resistant patients, the above results showed the potential use of Compound B alone or in combination with other agents to overcome venetoclax resistance in cancers such as AML and ALL.

### Example 3: The combinations of the present invention could synergistically inhibit the proliferation of RS4;11Bcl2-G101V-Flag cell lines

Method used was the cell viability CTG assay. RS4;11BCL2-G101V-Flag cell lines are RS4;11 cells carrying Bcl2-G101V-Flag mutation.

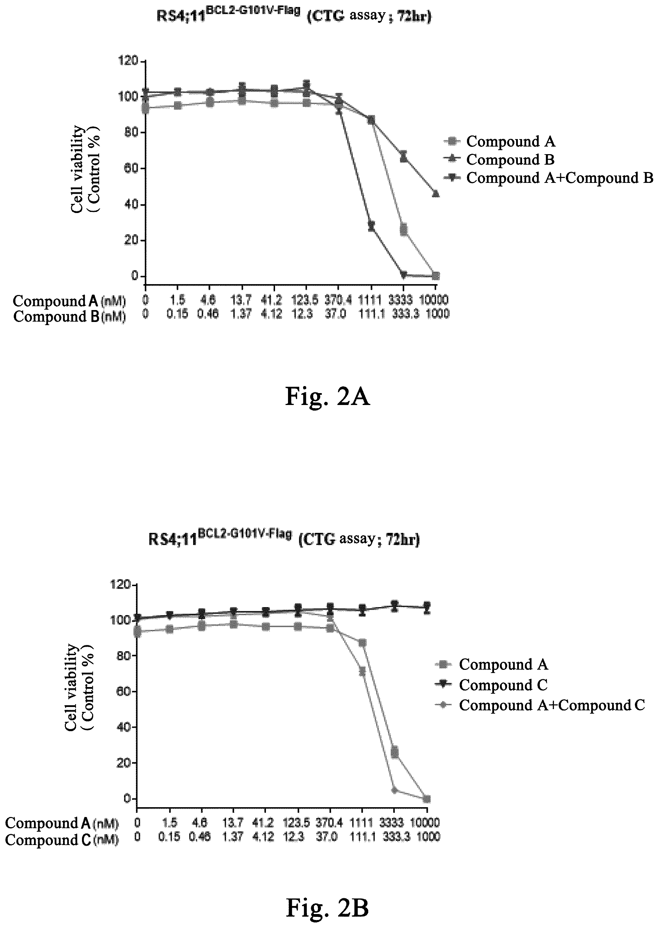

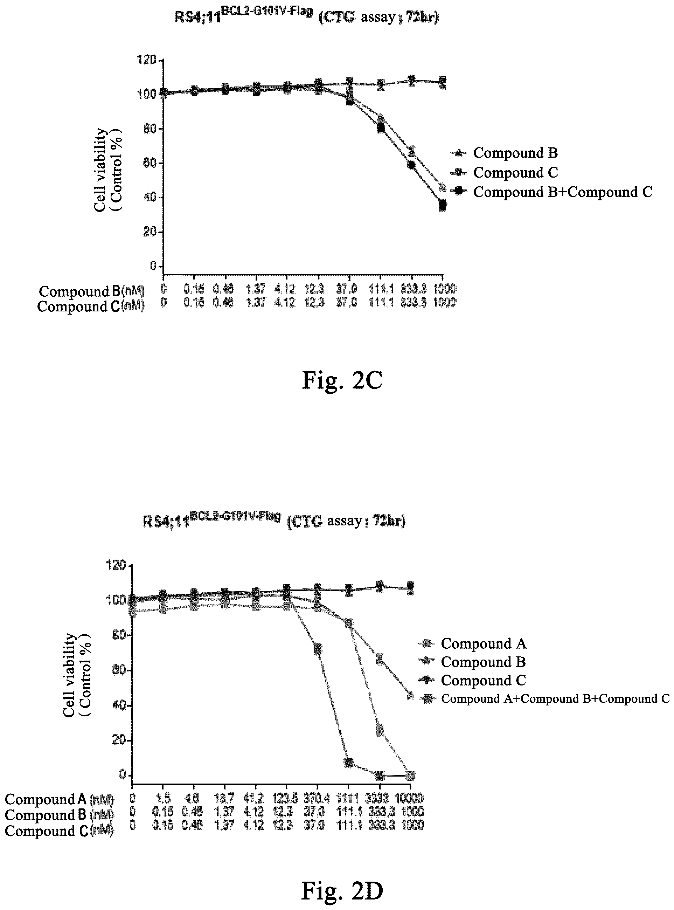

As shown in Figs. 2A-2D, in CTG assays (72h), the combination of Compound A and Compound B achieved lower cell viability than Compound A or Compound B alone; the combination of Compound A and Compound C achieved lower cell viability than Compound A or Compound C alone; the combination of Compound B and Compound C achieved lower cell viability than Compound B or Compound C alone; and the triple combination of Compound A, Compound B and Compound C achieved lower cell viability than Compound A, Compound B or Compound C alone.

### Conclusion:

The combinations of the present invention (Compound A+Compound B; Compound A+Compound C; and Compound B+Compound C) showed enhanced antiproliferative activity for RS4;11 Bcl2-G101V-Flag cell lines. Since BCL2 mutations are widely reported in venetoclax-resistant patients, the above results showed the potential use of the combinations of the present invention to overcome venetoclax resistance in cancers such as AML and ALL.

### Example 4: The combinations of the present invention could synergistically inhibit the proliferation of RS4;11Bcl2-V156D-Flag cell lines

Method used was the cell viability CTG assay. RS4;11BCL2-V156D-Flag cell lines are RS4;11 cells carrying Bcl2-V156D -Flag mutation.

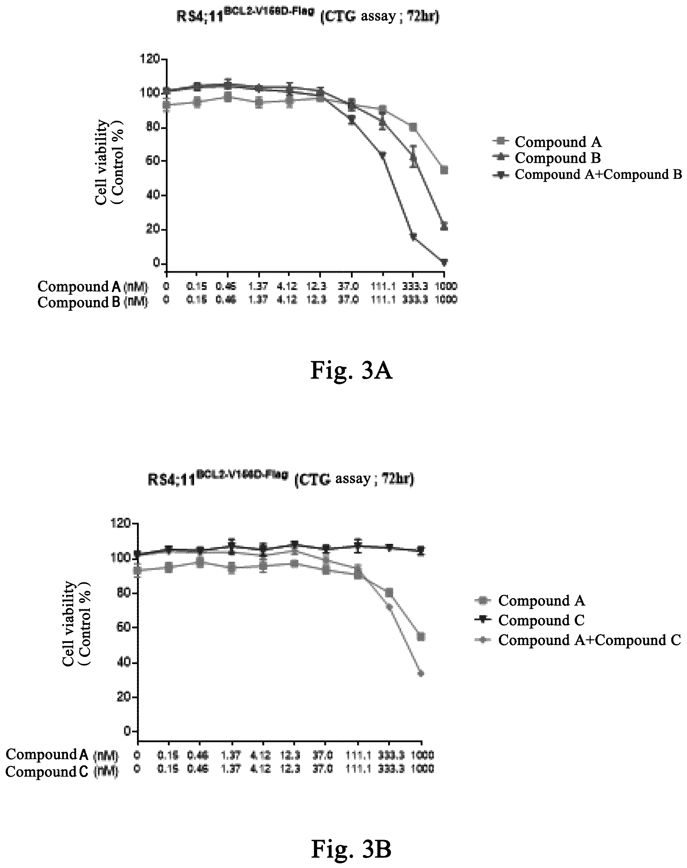

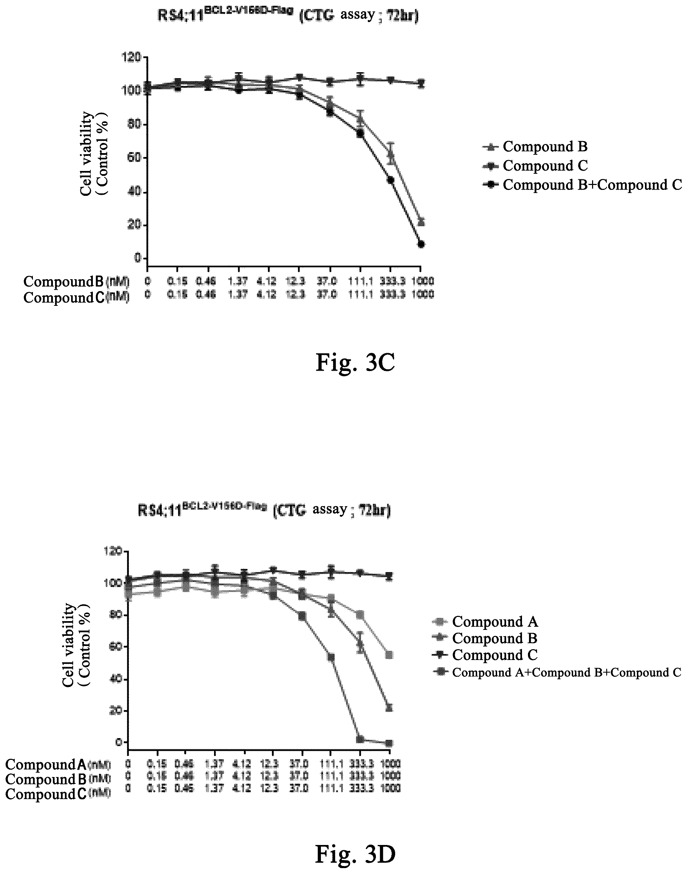

As shown in Figs. 3A-3D, in CTG assays (72h), the combination of Compound A and Compound B achieved lower cell viability than Compound A or Compound B alone; the combination of Compound A and Compound C achieved lower cell viability than Compound A or Compound C alone; the combination of Compound B and Compound C achieved lower cell viability than Compound B or Compound C alone; and the triple combination of Compound A, Compound B and Compound C achieved lower cell viability than Compound A, Compound B or Compound C alone.

### Conclusion:

The combinations of the present invention (Compound A+Compound B; Compound A+Compound C; and Compound B+Compound C) showed enhanced antiproliferative activity for RS4;11 Bcl2-V156D -Flag cell lines. Since BCL2 mutations are widely reported in venetoclax-resistant patients, the above results showed the potential use of the combinations of the present invention to overcome venetoclax resistance in cancers such as AML and ALL.

### Example 5: Co-targeting BCL-2, MDM2 and IAP is most effective in Compound A induced resistant cell lines (AML and ALL)

Method used was the cell viability CTG assay. Compound A induced resistant cell lines (MV-4-11; RS4;11; and MOLM-13) were induced by chronically treating cells with AG-2575 before the tests.

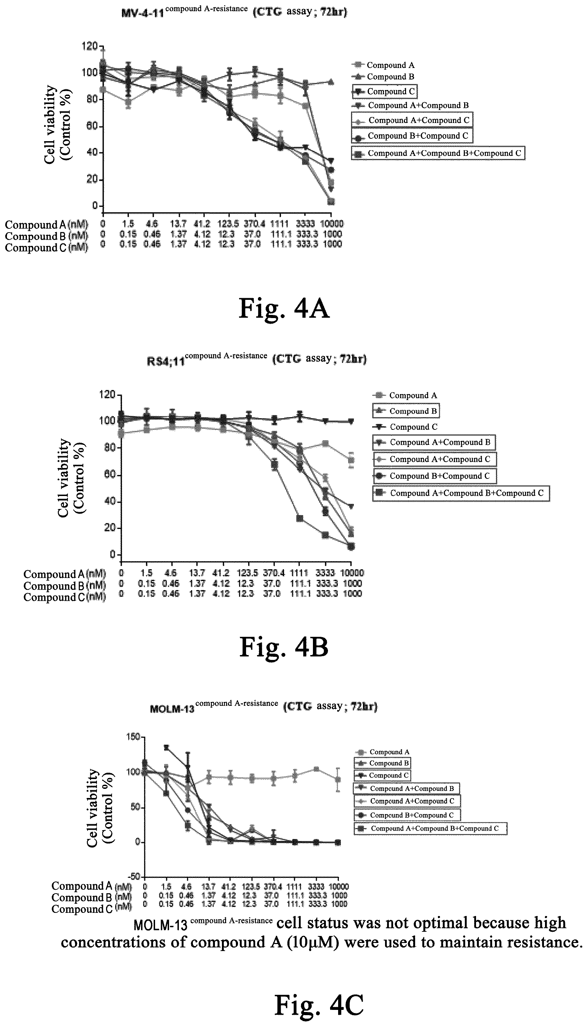

As shown in Figs. 4A-4C, in CTG assays (72h), the triple combination of Compound A, Compound B and Compound C achieved the lowest cell viability.

### Conclusion:

The triple combination of Compound A (a Bcl-2 inhibitor), Compound B (a MDM2 inhibitor) and Compound C (an IAP inhibitor) showed the best antiproliferative activity for Compound A induced resistant cell lines (MV-4-11; RS4;11; and MOLM-13). Thus, co-targeting BCL-2, MDM2 and IAP is most effective in Compound A induced resistant cell lines (AML and ALL). The above results showed the potential use of the triple combination to overcome Compound A resistance in cancers such as AML and ALL.

### Example 6: The combination of Compound A and Compound B overcomes venetoclax-resistance in tumor models carrying BCL-2 mutations

Method used was the subcutaneous xenograft assay. RS4;11BCL2-V156D-Flag cell lines were RS4;11 cells carrying Bcl2-V156D -Flag mutation.

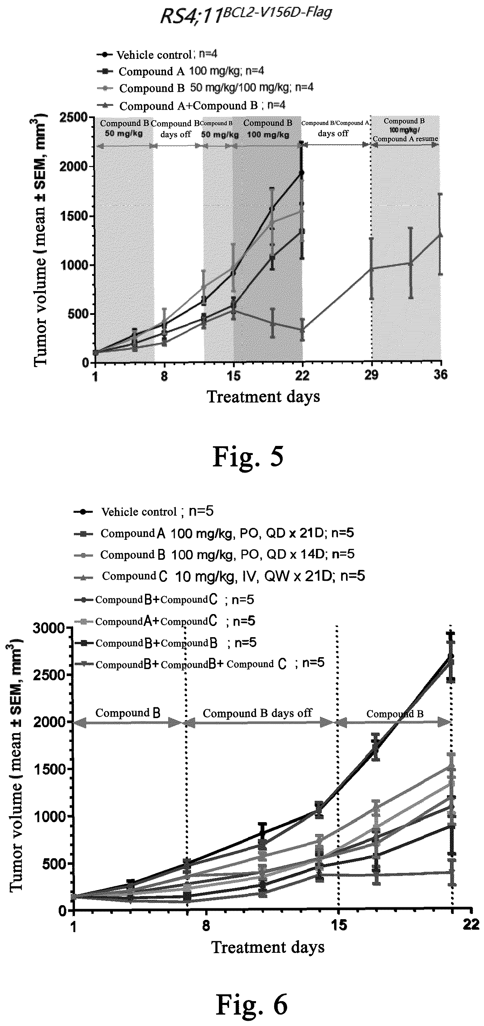

As shown in Fig. 5, in subcutaneous xenograft assays, tumor growth curve indicates that the combination of Compound A and Compound B is superior to each single-agent in suppressing tumor growth (based on the tumor volume).

### Conclusion:

The combination of Compound A and Compound B is superior to each single-agent in suppressing tumor growth. Since Bcl2-V156D is a mutation reported in venetoclax-resistant patients, the above results showed the potential use of the combination of Compound A and Compound B to overcome venetoclax resistance in cancers such as AML and ALL.

### Example 7: Combination treatments in RS4;11^{BCL-2-G101V-Flag} human ALL cancer xenograft model in mice

Method used was the subcutaneous xenograft assay. RS4;11BCL2-G101V-Flag cell lines were RS4;11 cells carrying Bcl2-G101V -Flag mutation.

As shown in Fig. 6, in subcutaneous xenograft assays, the combination of Compound A and Compound B, the combination of Compound A and Compound C, the combination of Compound B and Compound C and the triple combination of Compound A, Compound B and Compound C achieved lower tumor growth than each single-agent (based on the tumor volume). Specifically, the triple combination of Compound A, Compound B and Compound C achieved the lowest tumor growth.

### Conclusion:

The combinations of the present invention (Compound A + Compound B; Compound A + Compound C; Compound B + Compound C and Compound A + Compound B + Compound C) showed enhanced antiproliferative activity for RS4;11^{BCL-2-G101V-Flag} human ALL cancer xenograft model in mice. Since BCL2 mutations (including BCL2-G101V mutation) are widely reported in venetoclax-resistant patients, the above results showed the potential use of the combinations of the present invention to overcome venetoclax resistance in cancers such as AML and ALL.

### Example 8: Compound A + Compound B + Aza vs. Compound A + Compound B + Compound C in RS4;11-VEN-R human ALL cancer xenograft model in mice

Method used was the xenograft assay. RS4;11-VEN-R human ALL cancer xenograft model in mice was established in the assay. The model was venetoclax (VEN) resistant.

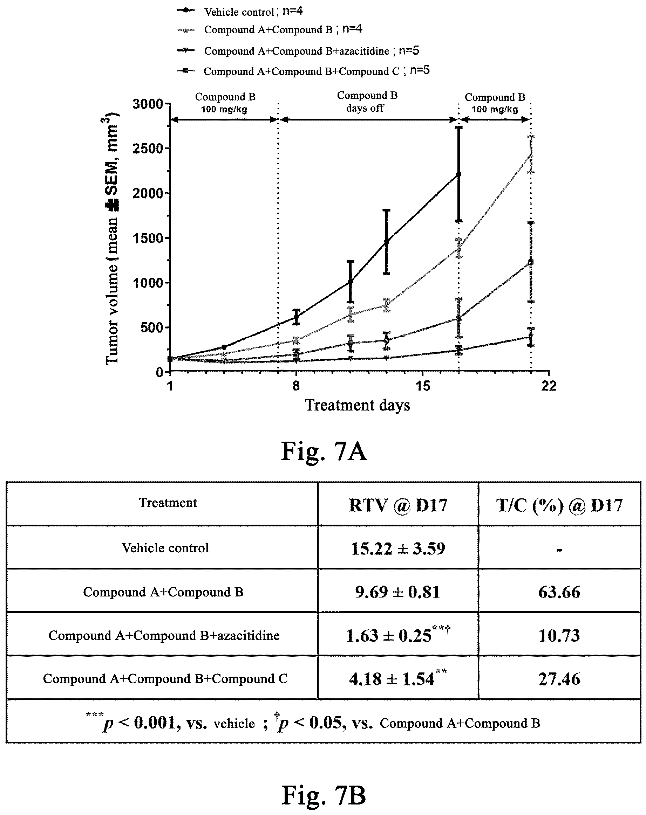

As shown in Figs. 7A and 7B, in xenograft assays, the combination of Compound A and Compound B achieved the decreased tumor volume as compared with the vehicle control. Further, the triple combination of Compound A and Compound B and Azacitidine and the combination of Compound A and Compound B and Compound C are superior to the combination of Compound A and Compound B and the vehicle control. Specifically, the antiproliferative activity of the combination of Compound A and Compound B and Compound C is comparable to that of the combination of Compound A and Compound B and Azacitidine.

### Conclusion:

The combination of Compound A and Compound B showed antiproliferative activity for RS4;11-VEN-R human ALL cancer xenograft model, and the further inclusion of Compound C could enhance the antiproliferative activity. The above results showed the potential use of the combinations of the present invention to overcome venetoclax resistance (VEN-R) in cancers such as AML and ALL.

### Example 9: The combination effect of Compound A+Compound C in AML cells (72 hr)

Method used was the cell viability WST assay. MOLM-13 WAT assay and MV-4-11 WST assay were respectively conducted.

The combination index (CI) method was based on that described by Chou and Talalay (Chou TC. Drug combination studies and their synergy quantification using the Chou-Talalay method. Cancer Res. 2010 Jan 15;70(2):440-6. doi: 10.1158/0008-5472.CAN-09-1947), and the CI value was calculated by using CalsuSyn software.

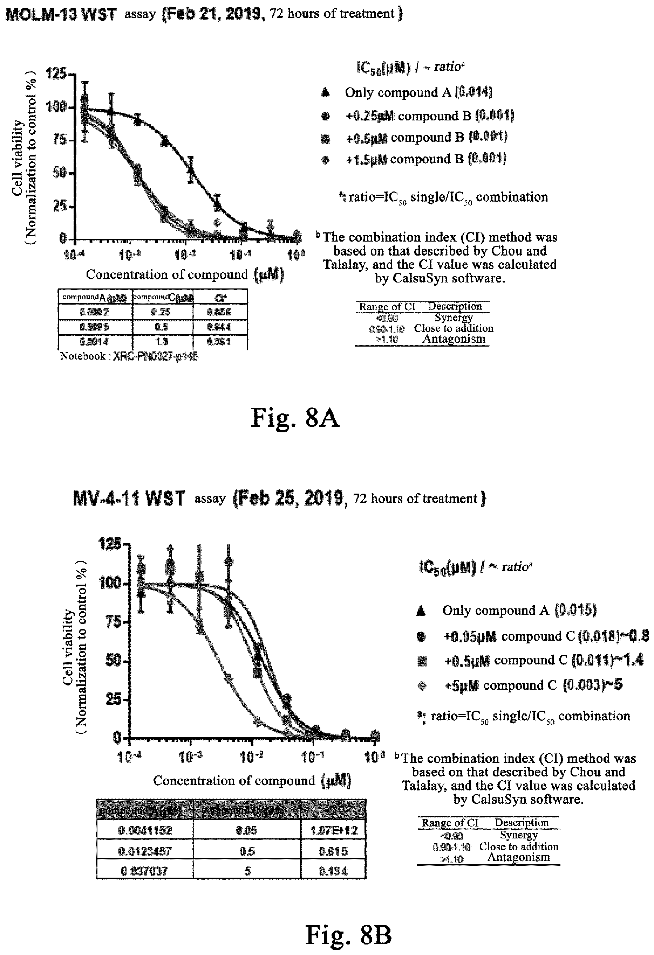

As shown in Figs. 8A and 8B, in cell viability WST assays, synergy between Compound A and Compound C in AML cell lines MOLM-13 and MV-4-11 was demonstrated based on the CI values. Specifically, CI value <0.9 indicates synergy.

### Conclusion:

The combination of Compound A and Compound C achieved synergy in AML cell lines MOLM-13 and MV-4-11. The above results showed the potential use of the combination of Compound A and Compound C to treat cancers such as AML.

### Example 10: The combination treatment with Compound A and Compound C in subcutaneous MV-4-11 AML achieves synergy

Method used was the subcutaneous xenograft assay. Subcutaneous MV-4-11 AML model was used in this Example.

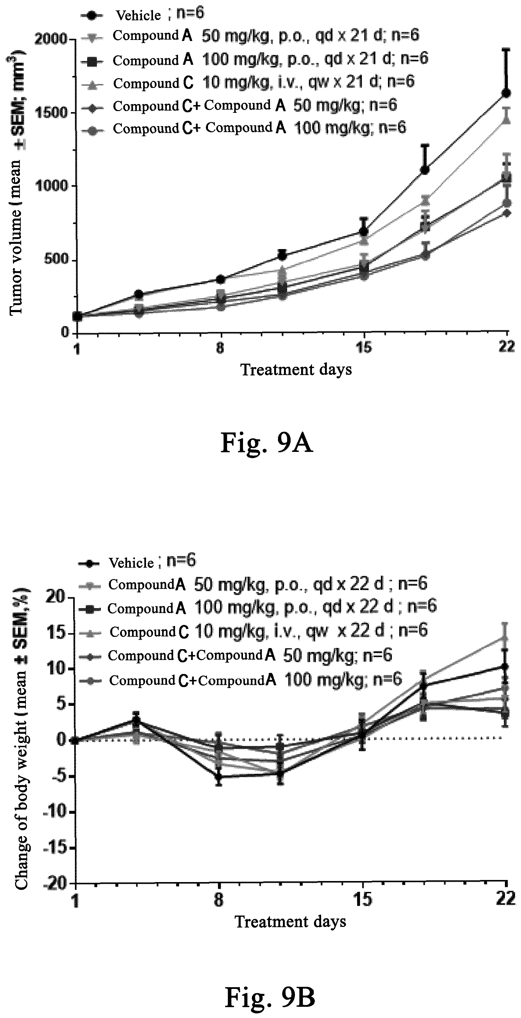

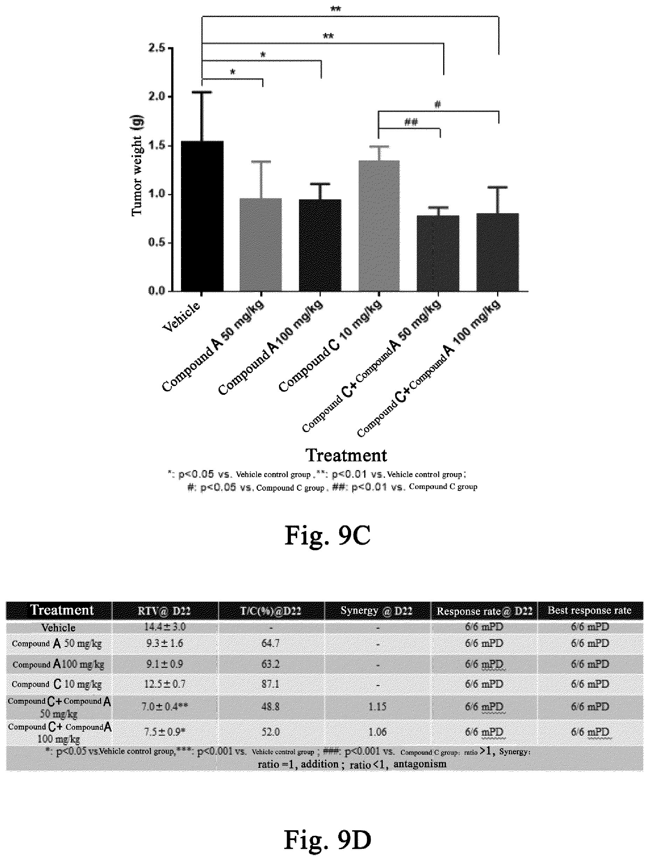

As shown in Figs. 9A-9D, synergy between Compound A and Compound C in AML MV-4-11 xenograft model was demonstrated by the enhanced tumor suppression reflected by T/C and tumor voloume. Further, synergy ratio of >1 also indicated synergisitic antitumor activity.

### Conclusion:

The combination of Compound A and Compound C achieved synergy in AML MV-4-11 xenograft model. The above results showed the potential use of the combination of Compound A and Compound C to treat cancers such as AML.

### Example 11: The combination treatment with Compound A and Compound C in orthotopic MOLM-13-luc human AML model

Method used was the orthotopic/systemic xenograft assay. Orthotopic MOLM-13-luc human AML model was used in this Example.

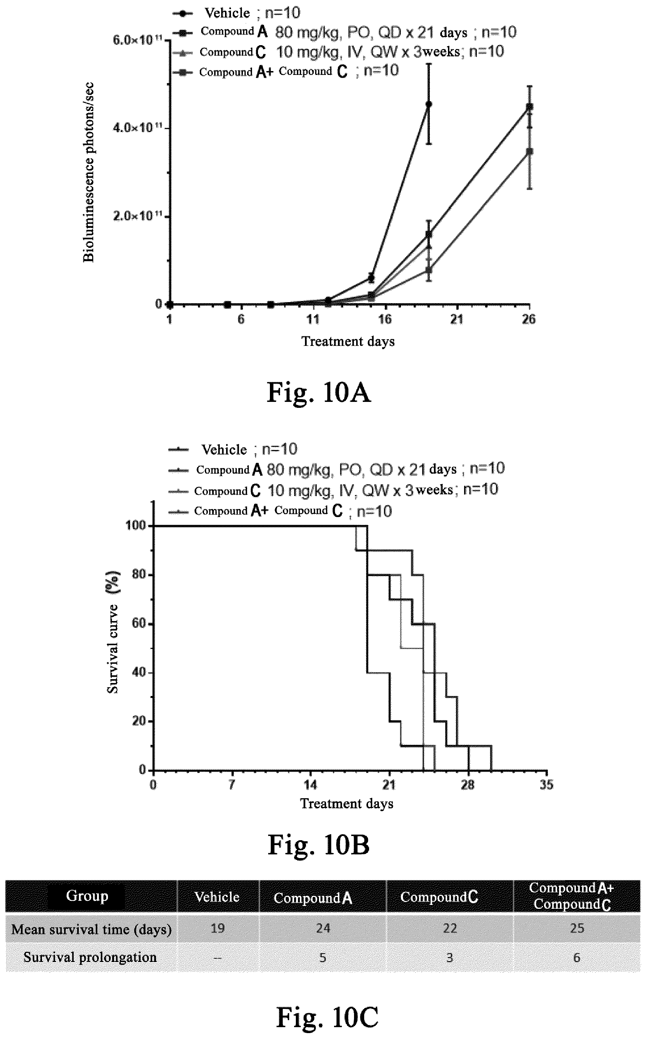

Reference is made to Figs. 10A-10C, Fig. 10A showed bioluminescence representing tumor burden of animals; Fig. 10B showed the survival curve; and Fig. 10C showed the Median survival time and the Survival prolongation. As shown in Figs. 10A-10C, the combination treatment with Compound A and Compound C demonstrated benefit over each single agent in the orthotopic MOLM-13-luc human AML model.

### Conclusion:

The combination of Compound A and Compound C achieved benefit in the orthotopic MOLM-13-luc human AML model. The above results showed the potential use of the combination of Compound A and Compound C to treat cancers such as AML.

### Example 12: The combination treatment with Compound A, Compound B and/or Compound C in p53 WT systemic OCI-AML 3 AML model (intrinsic resistant to venetoclax)

Method used was the orthotopic/systemic xenograft assay. The p53 WT systemic OCI-AML 3 AML model was used in this Example, which was a model intrinsic resistant to venetoclax.

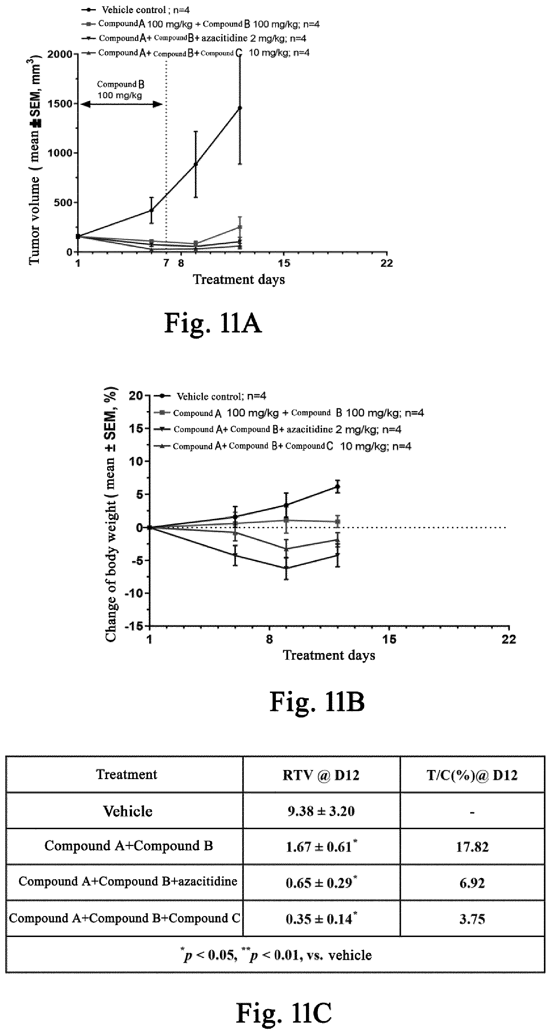

Reference is made to Figs. 11A-11C, Fig. 11A showed the tumor volume, Fig. 11B showed the body weight change; and Fig. 11C showed the RTV and T/C values. As shown in Figs. 11A-11C, the combination treatment with Compound A+Compound B or Compound A+Compound B+Compound C demonstrated benefit over vehicle control in the p53 WT systemic OCI-AML 3 AML model.

### Conclusion:

The combination of Compound A and Compound B and the combination of Compound A and Compound B and Compound C achieved benefit in the p53 WT systemic OCI-AML 3 AML model (intrinsic resistant to venetoclax). The above results showed the potential use of the combination in the present invention to treat cancers such as AML resistant to venetoclax.

### Example 13: Co-targeting Intrinsic and Extrinsic Apoptosis to Maximize Cell Death Induction in Venetoclax-resistant AML Cells

the inventors treated Molm13 cells lacking TP53 or carrying TP53 mutations (R248W, KO, R175H, R282W, Y220C, R248Q) generated by CRISPR (Boettcher S et al., Science 2019) with the three agents and their combinations. As shown in 12A-12K, all mutant cells were insensitive to single drugs. Enhanced activity was observed when any of two agents were combined and combined inhibition of Bcl-2, IAPs, and MDM2 most effectively induced cell death in TP53 knockout and all TP53 mutant cells (P *<* 0.05 for the triple combination compared to any of the double combinations or single agent treatments, and double combinations compared to their respective single agent treatments). Western blot analysis showed that decreased cIAP1, cIAP2, XIAP, or p21 was observed in single agent or combination-treated cells. Only in the triple combination group, cIAP1, cIAP2, and XIAP as well as MDM2 were largely diminished and p21 was marked decreased.

In conclusion, the above study demonstrates that co-targeting intrinsic and extrinsic apoptosis maximizes cell death induction in AML cells with acquired resistance to VEN or with TP53 mutations by antagonizing Bcl-2, eliminating clAPs and XIAP, as well as MDM2 and p21.

### Example 14: Compound E shows single agent activity in BCL-2 inhibitor(BCL-2i) insensitive cells RPMI 8226

Method used was the cell viability CTG assay. BCL-2i insensitive multplie myeloma (MM) model RPMI 8226 cells were established, its IC₅₀ to Compound A (BCL-2i) was 5.58 uM (insensitive).

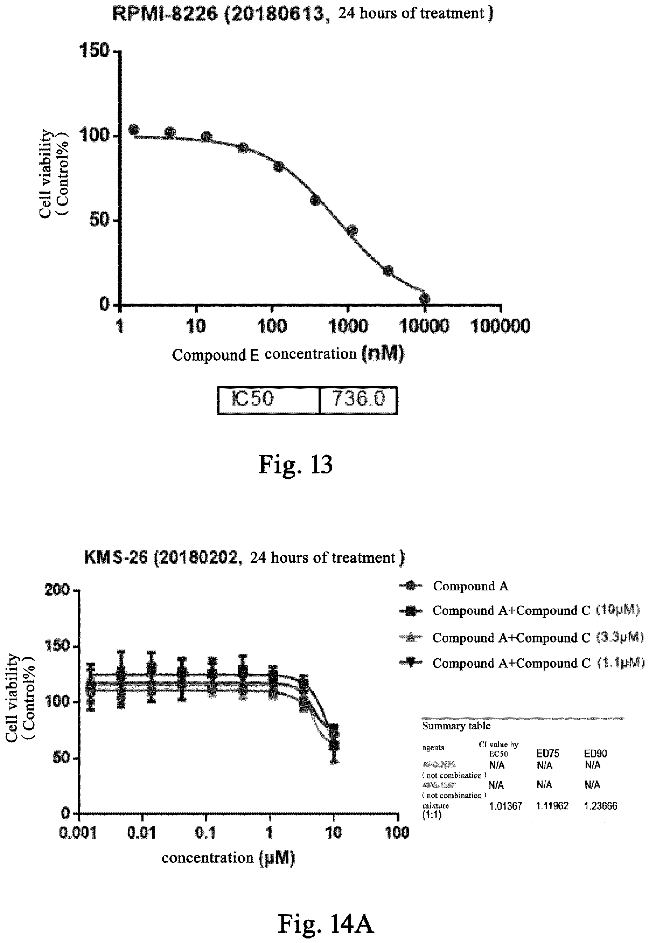

As shown in Fig. 13, in MM cells that are insensitive to BCL-2 inhibitor (including venetoclax), Compound E showed single agent activity in inhibiting cell proliferation (RPMI 8226 model).

### Conclusion:

Compound E showed single agent activity in BCL-2i insensitive cells RPMI 8226, the above result showed the potential use of Compound E alone or in combination with other agents to overcome venetoclax or Compound A resistance in cancers such as MM.

### Example 15: Compound E and Compound C combination synergistically sensitizes BCL-2i resistant cells to proliferation inhibition

Method used was the cell viability CTG assay. BCL-2i resistant MM model KMS-26 was established.

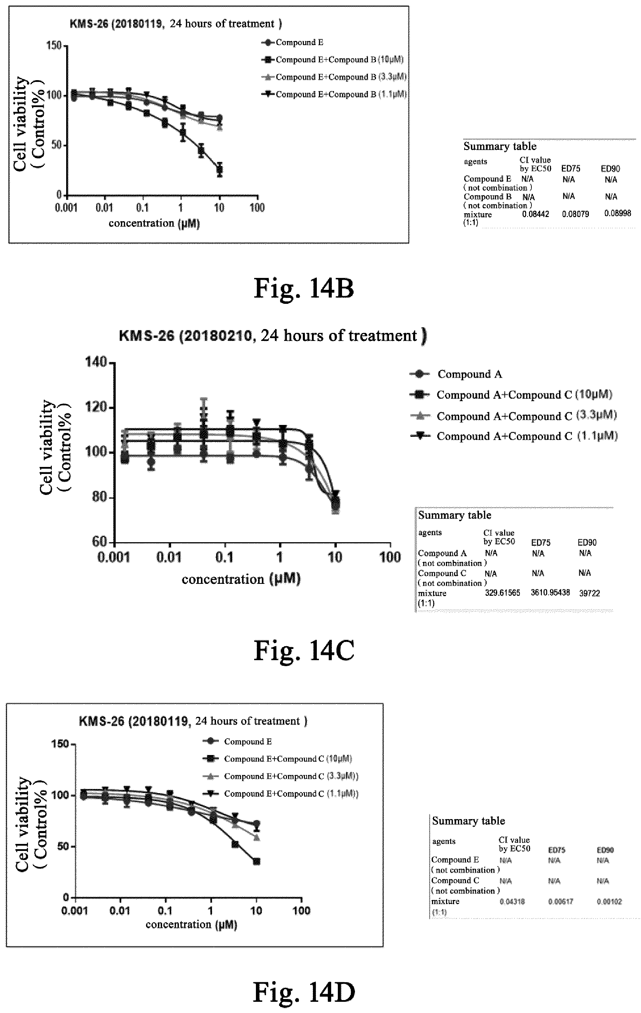

As shown in Figs. 14A-14D, in MM cells insensitive or resistant to BCL-2 inhibitor (including venetoclax), Compound E in combination with Compound B(MDM2 inhibitor) or Compound C(IAP inhibitor) sensitized cells to proliferation inhibition (KMS-26 cell lines).

### Conclusion:

Compound E and Compound C/Compound B combination synergistically sensitized BCL-2i insensitive cells to proliferation inhibition, the above result showed the potential use of Compound E in combination with other agents to overcome venetoclax or Compound A resistance in cancers such as MM.

### Example 16: Compound C shows single agent activity in MM cells insensitive to BCL-2 inhibitor

Method used was the cell viability CTG assay. BCL-2i resistant MM mode NCI-H929 was established.

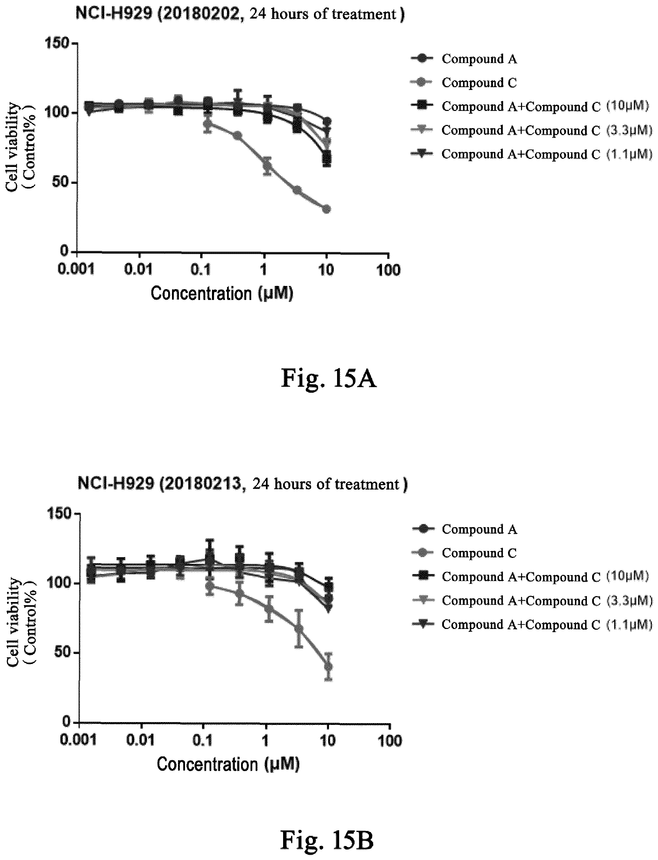

As shown in Figs. 15A and 15B, in MM cells insensitive or resistant to BCL-2 inhibitor (including venetoclax), Compound C showed single agent activity to inhibit cell proliferation (NCI-H929 model).

### Conclusion:

Compound C showed single agent activity in MM cells insensitive to BCL-2 inhibitor, the above result showed the potential use of Compound C alone or in combination with other agents to overcome venetoclax or Compound A resistance in cancers such as MM.

### Example 17: Compound E/ Compound D showed single drug activity in Venetoclax-resistant MM cells and xenotransplantation models.

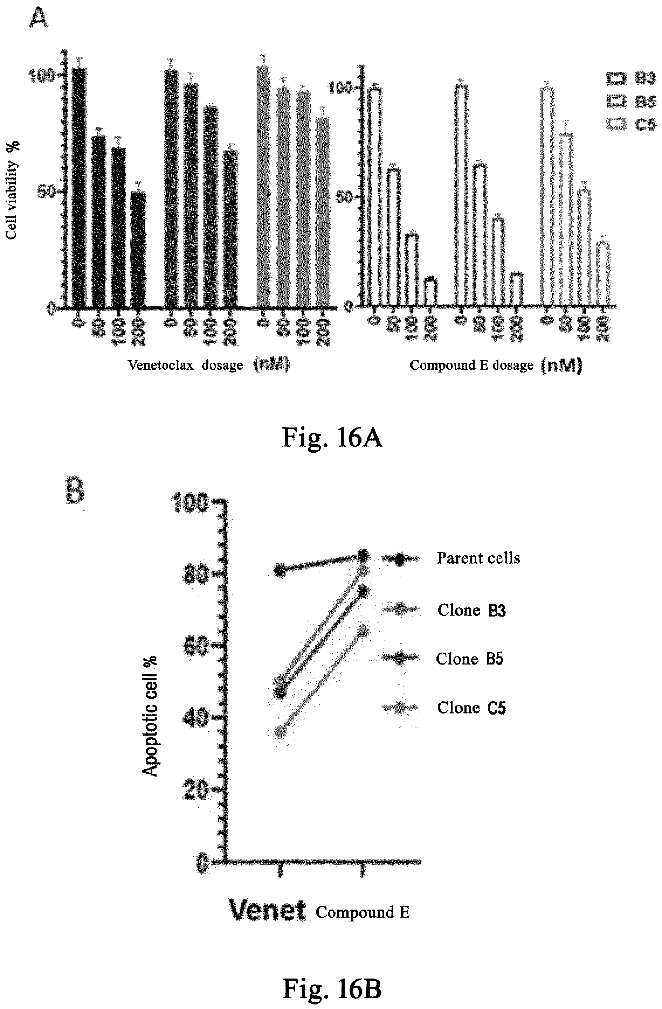

KMS27 sensitive MM cell line was treated with high concentration of Venetoclax, and MM acquired drug resistance phenotype was established. Colonies produced by living cells were cultured in high-dose Venetoclax to produce monoclonal drug-resistant amplified persistence (DTEP) clones. Compared with the parent cells, IC50 is increased by at least 3 to 10 times. Compared with Venetoclax, BCL-2IBCL-xLIBCL-w inhibitor compound E successfully inhibited the proliferation of drug-resistant clones (Figure 16A) and induced apoptosis (Figure 16B).

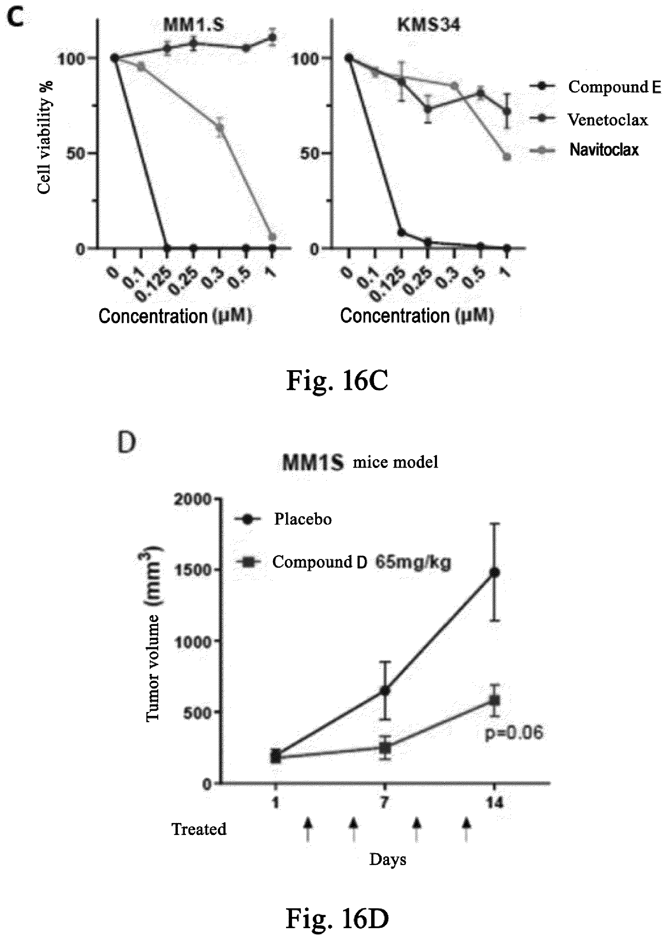

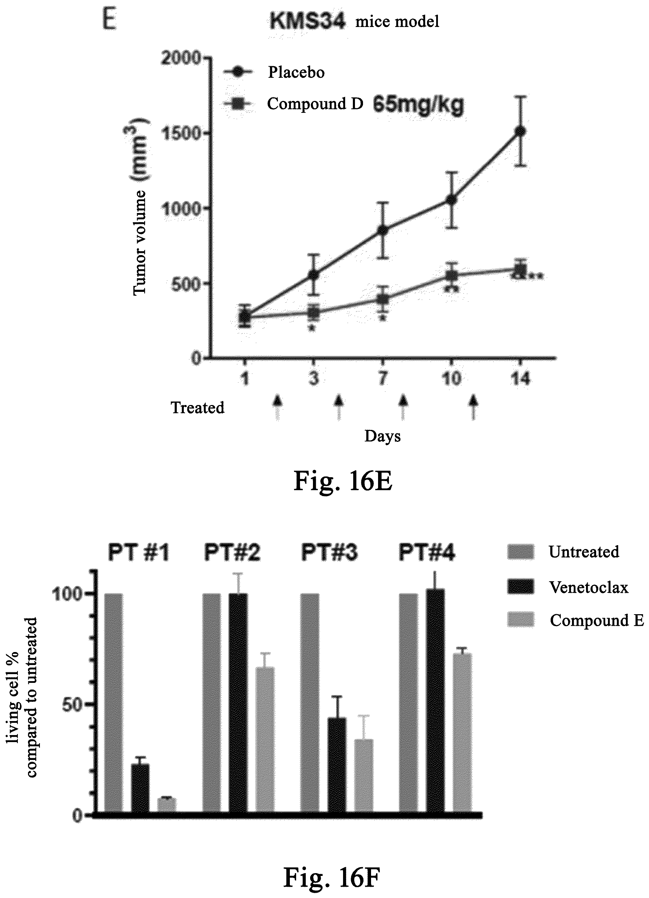

In order to explore whether compound E is also effective under the condition of intrinsic drug resistance of Venetokrax, we treated Venetokrax-resistant MM cell lines (MM1.S and KMS34) with Venetokrax, compound E or Navitoclax. As shown in fig. 16C, compound E is the most effective in inducing cell death. As shown in figs. 16D and 16E, compound D, a prodrug of Compound E with lower platelet toxicity, was also effective in CDX models of these two cell lines.

The activity of compound E was confirmed in primary MM cells in vitro. As shown in fig. 16F, we treated MM cells from 4 MM patients, and observed that the activity of Compound E was higher than venetoclax.

To sum up, compound E/ compound D showed single drug activity in Venetoclax-resistant MM cells and xenotransplantation models.

Having now fully described the methods, compounds, and compositions herein, it will be understood by those of skill in the art that the same can be performed within a wide and equivalent range of conditions, formulations, and other parameters without affecting the scope of the methods, compounds, and compositions provided herein or any embodiment thereof.

## Claims

1. A pharmaceutical combination comprising a Bcl-2 inhibitor and one or more anticancer reagents.

2. The pharmaceutical combination according to Claim 1, wherein the one or more anticancer reagents are selected from the group consisting of a MDM2 inhibitor, an IAP inhibitor, and other anticancer reagents.

3. The pharmaceutical combination according to Claim 1, wherein the one or more anticancer reagents are selected from the group consisting of a MDM2 inhibitor, an IAP inhibitor, and combination thereof.

4. The pharmaceutical combination according to any one of Claim 1-3, wherein the Bcl-2 inhibitor is a compound of Formula V: 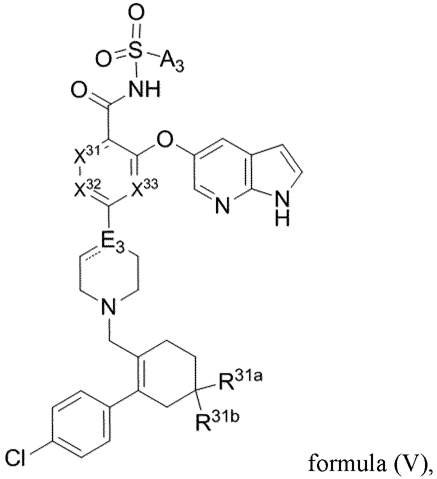 or a pharmaceutically acceptable salt or solvate thereof, wherein: 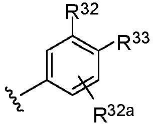
A₃ is selected from A-1 ;
E₃ is a nitrogen atom and -̅ -̅ -̅ is a single bond;
X³¹, X³², and X³³ are each independently selected from the group consisting of -CR³⁸ = and -N=;
R^{31a} and R^{31b} taken together with the carbon atom to which they are attached form a 3-, 4-, or 5-membered cycloalkyl;
R³² is selected from the group consisting of -NO₂, -SO₂CH₃, and -SO₂CF₃;
R^{32a} is selected from the group consisting of hydrogen and halogen;
R³³ is selected from -N(R^{34a})(R^{34b});
R^{34a} is selected from the group consisting of optionally substituted C₁₋₆ alkyl, optionally substituted C₃₋₆ cycloalkyl, heterocyclo, heteroalkyl, (cycloalkyl)alkyl, and (heterocyclo)alkyl;
R^{34b} is selected from the group consisting of hydrogen and C₁₋₄ alkyl;
R³⁸ is selected from the group consisting of hydrogen and halogen.

5. The pharmaceutical combination according to Claim 4, wherein the Bcl-2 inhibitor is selected from the group consisting of: 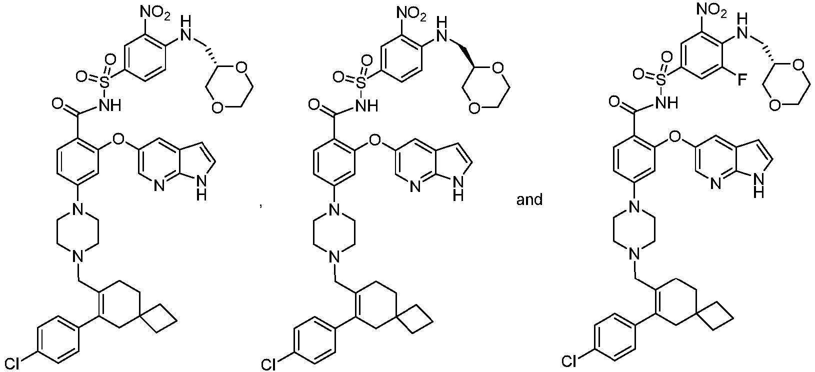 , or a pharmaceutically acceptable salt or solvate thereof

6. The pharmaceutical combination according to Claim 4 or 5, wherein the Bcl-2 inhibitor is: 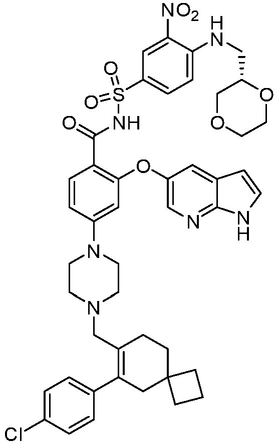 (S)-N-((4-(((1,4-dioxan-2-yl)methyl)amino)-3-nitrophenyl)sulfonyl)-2-((1H-pyrrolo[2,3-b]pyridin-5-yl)oxy)-4-(4-((6-(4-chlorophenyl)spiro[3.5]non-6-en-7-yl)methyl)piperazin-1-yl)benzamide (Compound A), or a pharmaceutically acceptable salt or solvate thereof.

7. The pharmaceutical combination according to any one of Claims 2 to 6, wherein the MDM2 inhibitor is a compound of formula (VI), or a pharmaceutically acceptable salt or solvate thereof: 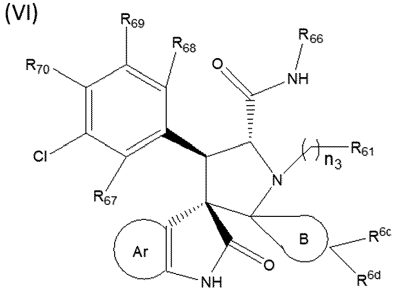 wherein 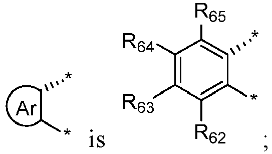
B is 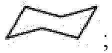 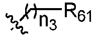
is H, CH₃, or CH₂CH₃
R₆₂, R₆₃, R₆₄, R₆₅, R₆₇, R₆₈, R₆₉, and R₇₀, independently, are selected from the group consisting of H, F, and Cl;
R₆₆ is 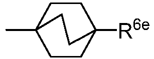 ; and
R^{6c} and R^{6d} are substituents on one carbon atom of ring B, wherein
R^{6c} is H, C₁₋₃ alkyl, or halo;
R^{6d} is H, C₁₋₃ alkyl, or halo;
R^{6e} is -C(=O)OR^{6a};
R^{6a} is hydrogen or unsubstituted C₁₋₄ alkyl.

8. The pharmaceutical combination according to Claim 7, wherein R₆₂ is H, R₆₃ is F or Cl, and R₆₄ and R₆₅ are H, R₆₇ is fluoro, each of R₆₈, R₆₉, and R₇₀ is H, R^{6c} is H, CH₃, or halo, and R^{6d} is H, CH₃, or halo.

9. The pharmaceutical combination according to Claim 7 or 8, wherein the MDM2 inhibitor is: 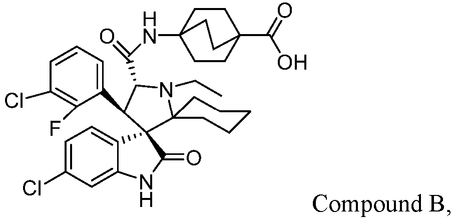 or a pharmaceutically acceptable salt or solvate thereof.

10. The pharmaceutical combination according to any one of Claims 2 to 9, wherein the IAP inhibitor is a compound of formula (I), or a pharmaceutically acceptable salt or solvate thereof: 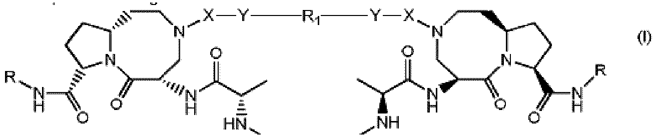 wherein
X is selected from 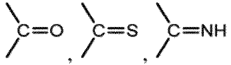 and -SO₂-;
Y is selected from -NH-, -O-, -S-, and absent;
R is selected from the group consisting of: 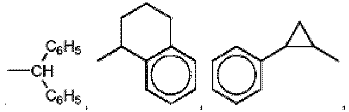 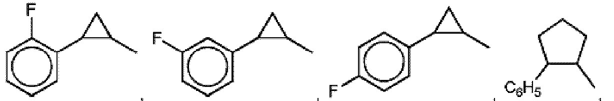 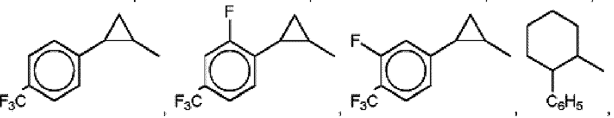 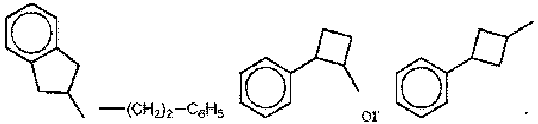
R₁ is selected from the group consisting of:

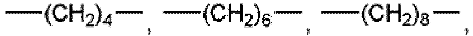

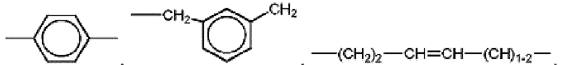

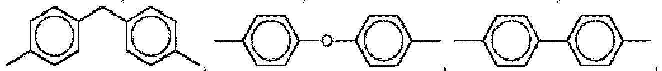

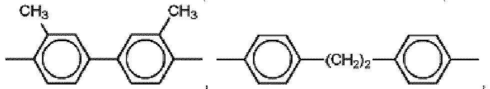

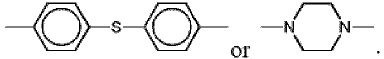

11. The pharmaceutical combination according to Claim 10, wherein the MDM2 inhibitor is: 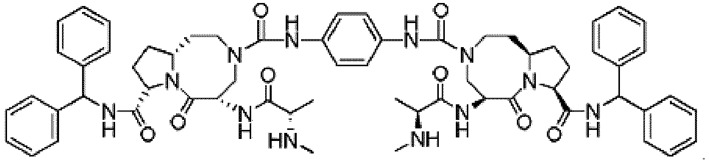 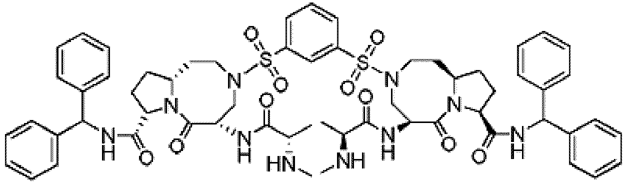 or 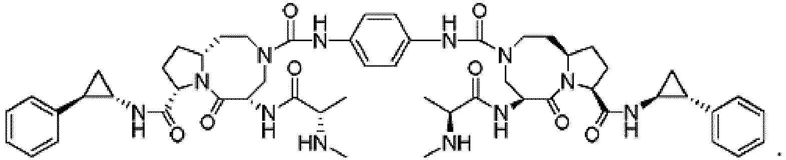 or a pharmaceutically acceptable salt or solvate thereof.

12. The pharmaceutical combination according to Claim 10 or 11, wherein the MDM2 inhibitor is: 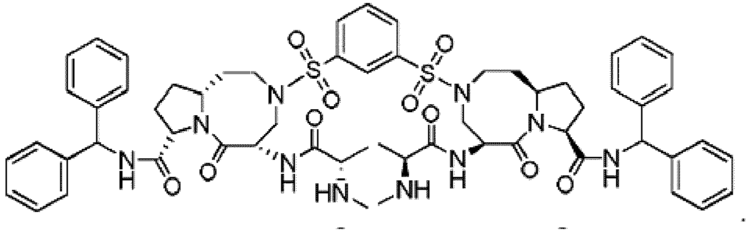 1,3-phenylenebis[7-(3S,5 S,9aR)-5-((S)-2-methylamino-propionamido)-3-diphenylcarbamyl-4-oxo-3a,7-diaza-decahydrocyclopentacyclooctene)]-sulfonamide (Compound C), or a pharmaceutically acceptable salt or solvate thereof.

13. The pharmaceutical combination according to any one of Claims 1 to 12 for use in treating or suppressing a cancer, reducing its severity, lowering its risk or inhibiting its metastasis in an individual, wherein the cancer is preferably selected from the group consisting of bladder cancer, breast cancer, cervical cancer, colon cancer (including colorectal cancer), esophageal cancer, esophageal squamous cell carcinoma, head and neck cancer, liver cancer, lung cancer (including small cell lung cancer, non-small cell lung cancer and lung squamous cell carcinoma), mesothelial tumor, melanoma, myeloma, rhabdomyosarcoma, inflammatory myofibroblastic tumor, neuroturbo chargeoma, pancreatic cancer, prostate cancer, kidney cancer, renal cell carcinoma, sarcoma (including osteosarcoma), skin cancer, squamous cell carcinoma, spindle cell carcinoma, gastric cancer, testicular cancer, thyroid cancer, uterine cancer, mesothelioma, neuroblastoma, cholangiocarcinoma, leiomyosarcoma, liposarcoma, nasopharyngeal carcinoma, neuroendocrine carcinoma, ovarian cancer, salivary gland cancer, metastasis caused by spindle cell carcinoma, anaplastic large cell lymphoma, thyroid undifferentiated carcinoma, non-Hodgkin's lymphoma, Hodgkin's lymphoma, and hematological malignancies, such as acute myeloid leukemia (AML), acute lymphoblastic leukemia (ALL), diffuse large B-cell lymphoma (DLBCL), follicular lymphoma (FL), chronic lymphocytic leukemia (CLL), chronic myeloid leukemia (CML), multiple myeloma (MM), uveal melanoma, pleural mesothelioma, peritoneal mesothelioma;
further preferably, the cancer is acute myeloid leukemia (AML), acute lymphoblastic leukemia (ALL) or multiple myeloma (MM).

14. The pharmaceutical combination according to claim 13, wherein the cancer is resistant or insensitive to Bcl-2 inhibitors such as compound A or venetoclax, further preferably, the cancer is acute myeloid leukemia (AML), acute lymphoblastic leukemia (ALL) or multiple myeloma (MM) resistant or insensitive to Bcl-2 inhibitors such as compound A or venetoclax.

15. The pharmaceutical combination according to claim 13 or 14, wherein the cancer is carrying Bcl-2 mutation (e.g., G101V, D103E, V156D and combination thereof) and/or TP53 mutation (e.g., R248Q, R175H, R282W, Y220C and combination thereof), further preferably, the cancer is acute myeloid leukemia (AML), acute lymphoblastic leukemia (ALL) or multiple myeloma (MM) carrying Bcl-2 mutation and/or TP53 mutation.

16. The pharmaceutical combination according to any one of Claims 1 to 15, wherein the weight ratio between the Bcl-2 inhibitor and the one or more anticancer reagents is 0.005-5000 : 0.005-5000, for example, 0.05-1500 : 0.005-5000, 0.1-6 : 0.005-4, 100 : 0.5-400, 100 : 1-350, 100 : 2-300, 100 : 5-200, 100 : 10-150, 100 : 10-100, 100 : 10-90, or 100: 20-80.

17. The pharmaceutical combination according to any one of Claims 1 to 15, wherein the molar ratio between the Bcl-2 inhibitor and the one or more anticancer reagents is 10-1: 1-10, for example, 10:1, 9:1, 8:1, 7:1, 6:1, 5:1, 4:1, 3:1, 2:1, 1:1: 1:2, 1:3, 1:4, 1:5, 1:6, 1:7, 1:8, 1:9, 1:10, and the ranges between any of the aforementioned values are also included.

18. The pharmaceutical combination according to any one of Claims 2-17, wherein the Bcl-2 inhibitor is: 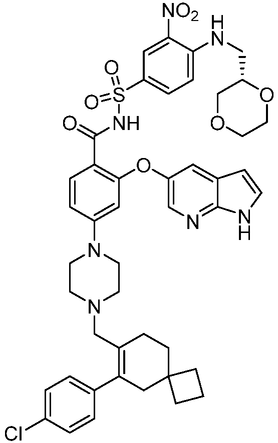 (S)-N-((4-(((1,4-dioxan-2-yl)methyl)amino)-3-nitrophenyl)sulfonyl)-2-((1H-pyrrolo[2,3-b]pyridin-5-yl)oxy)-4-(4-((6-(4-chlorophenyl)spiro[3.5]non-6-en-7-yl)methyl)piperazin-1-yl)benzamide (Compound A), or a pharmaceutically acceptable salt or solvate thereof; and/or
the MDM2 inhibitor is: 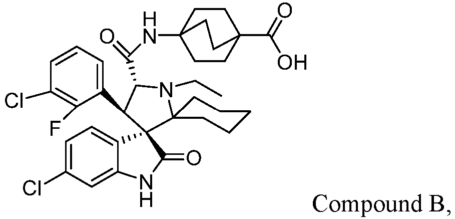
or a pharmaceutically acceptable salt or solvate thereof; and/or
the MDM2 inhibitor is: 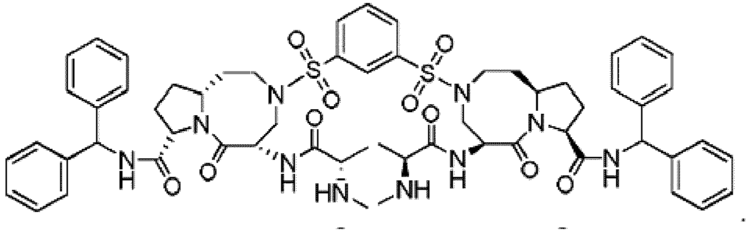 1,3-phenylenebis[7-(3S,5 S,9aR)-5-((S)-2-methylamino-propionamido)-3-diphenylcarbamyl-4-oxo-3a,7-diaza-decahydrocyclopentacyclooctene)]-sulfonamide (Compound C), or a pharmaceutically acceptable salt or solvate thereof.

19. A pharmaceutical composition comprising the pharmaceutical combination according to any one of Claims 1 to 18, and optionally a pharmaceutically acceptable carrier.

20. The pharmaceutical composition according to Claim 19, which is in the form of a tablet, a capsule, a granule, a syrup, a powder, a lozenge, a sachet, a cachet, an elixir, a suspension, an emulsion, a solution, a syrup, an aerosol, an ointment, a cream and an injection.

21. A method for treating or suppressing a cancer, reducing its severity, lowering its risk or inhibiting its metastasis in an individual, comprising administering to the individual a therapeutically effective amount of a Bcl-2 inhibitor, and optionally a therapeutically effective amount of one or more anticancer reagents;
preferably, the Bcl-2 inhibitor inhibitor is as defined in any one of Claims 4 to 6 and the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

22. The method according to Claim 21, wherein the Bcl-2 inhibitor is administrated in an amount of from about 0.005 mg/day to about 5000 mg/day, such as an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

23. The method according to Claim 21 or 22 wherein the Bcl-2 inhibitor is administrated in an amount of from about 1 ng/kg to about 200 mg/kg, about 1 µg/kg to about 100 mg/kg, or about 1 mg/kg to about 50 mg/kg per unit dose, for example, administrated in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg per unit dose, and administrated with one or more (e.g., 1, 2, 3, 4, 5, 6, 7, 8, 9 or 10) unit doses per day.

24. The method according to any one of Claims 21 to 23, wherein the one or more anticancer reagents are administrated in an amount of from 0.005 mg/day to about 5000 mg/day, for example, about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

25. The method according to any one of Claims 21 to 24, wherein the one or more anticancer reagents are administrated in an amount of from about 1 ng/kg to about 200 mg/kg, from about 1 µg/kg to about 100 mg/kg, or from about 1 mg/kg to about 50 mg/kg per unit dose, for example, administrated in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg /kg, about 70 mg/kg, about 80 mg/kg, about 90 mg/kg, about 100 mg/kg, about 125 mg/kg, about 150 mg/kg, about 175 mg/kg, about 200 mg/kg per unit dose, and administered with one or more (e.g., 1, 2, 3, 4, 5, 6, 7, 8, 9, or 10) unit doses per day.

26. The method according to any one of Claims 21 to 25, wherein the Bcl-2 inhibitor, and the one or more anticancer reagents are administered together, simultaneously, sequentially or alternately.

27. The method according to any one of Claims 21 to 26, wherein the Bcl-2 inhibitor, and the one or more anticancer reagents are administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 28 days, at least 30 days, at least 35 days, at least 40 days, at least 45 days, or at least 50 days.

28. The method according to any one of Claims 21 to 27, wherein the Bcl-2 inhibitor, and the one or more anticancer reagents are administered for one or more (e.g., 1, 2, 3, 4, 5, 6, 7, 8, 9 or 10) courses of treatment, in which each of the courses lasts at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days; and there is an interval of 0, 1, 2, 3, 4, 5, 6, 7, 8, 9, 10 days, two weeks, three weeks or four weeks between every two courses of treatment.

29. The method according to any one of Claims 21 to 28, wherein the Bcl-2 inhibitor, and the one or more anticancer reagents are administrated via the same (e.g., oral) or different routes (e.g., oral and parenteral (e.g., injection), respectively).

30. Use of a Bcl-2 inhibitor in combination with one or more anticancer reagents for treating or suppressing a cancer, reducing its severity, lowering its risk or inhibiting its metastasis in an individual,
preferably, the Bcl-2 inhibitor inhibitor is as defined in any one of Claims 4 to 6 and the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

31. Use of a Bcl-2 inhibitor in combination with one or more anticancer reagents in the manufacture of a medicament for treating or suppressing a cancer, reducing its severity, lowering its risk or inhibiting its metastasis in an individual,
preferably, the Bcl-2 inhibitor inhibitor is as defined in any one of Claims 4 to 6 and the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

32. Use of a MDM2 inhibitor alone or in combination with one or more anticancer reagents for treating or suppressing a cancer, reducing its severity, lowering its risk or inhibiting its metastasis in an individual,
preferably, the MDM2 inhibitor inhibitor is as defined in any one of Claims 7 to 9 and the anticancer reagent is selected from the group consisting of a Bcl-2 inhibitor, an IAP inhibitor, and other anticancer reagents, such as those defined in any one of Claims 4 to 6 and 10 to 12, and
preferably, the cancer is as defined in any one of Claims 13-15.

33. Use of a MDM2 inhibitor alone or in combination with one or more anticancer reagents in the manufacture of a medicament for treating or suppressing a cancer, reducing its severity, lowering its risk or inhibiting its metastasis in an individual,
preferably, the MDM2 inhibitor inhibitor is as defined in any one of Claims 7 to 9 and the anticancer reagent is selected from the group consisting of a Bcl-2 inhibitor, an IAP inhibitor, and other anticancer reagents, such as those defined in any one of Claims 4 to 6 and 10 to 12, and
preferably, the cancer is as defined in any one of Claims 13-15.

34. A pharmaceutical combination for treating or suppressing a cancer, reducing its severity, lowering its risk or inhibiting its metastasis in an individual comprising a MDM2 inhibitor and one or more anticancer reagents,
preferably, the MDM2 inhibitor inhibitor is as defined in any one of Claims 7 to 9 and the anticancer reagent is selected from the group consisting of a Bcl-2 inhibitor, an IAP inhibitor, and other anticancer reagents, such as those defined in any one of Claims 4 to 6 and 10 to 12, and
preferably, the cancer is as defined in any one of Claims 13-15.

35. Use of a Bcl-2/Bcl-xL inhibitor alone or in combination with one or more anticancer reagents for treating or suppressing a cancer, reducing its severity, lowering its risk or inhibiting its metastasis in an individual,
preferably, the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

36. Use of a Bcl-2/Bcl-xL inhibitor alone or in combination with one or more anticancer reagents in the manufacture of a medicament for treating or suppressing a cancer, reducing its severity, lowering its risk or inhibiting its metastasis in an individual,
preferably, the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

37. A pharmaceutical combination for treating or suppressing a cancer, reducing its severity, lowering its risk or inhibiting its metastasis in an individual comprising a Bcl-2/Bcl-xL inhibitor and one or more anticancer reagents,
preferably, the anticancer reagent is selected from the group consisting of a MDM2 inhibitor, an IAP inhibitor, and other anticancer reagents, such as those defined in any one of Claims 7 to 12, and
preferably, the cancer is as defined in any one of Claims 13-15.

38. The use of Claim 35 or 36 or the pharmaceutical combination of claim 37, wherein the Bcl-2/Bcl-xL inhibitor is selected from: 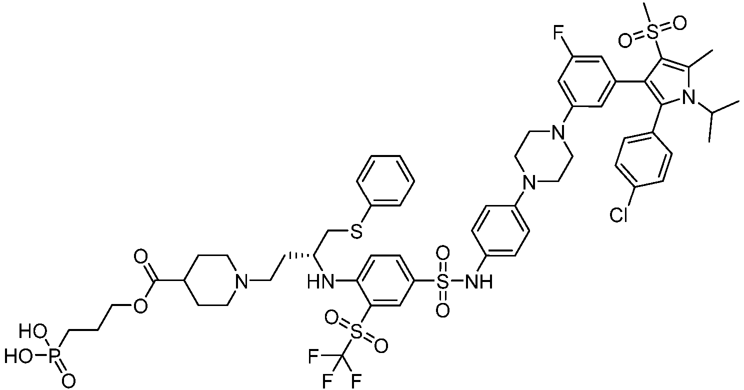
(3R)-1-(3-(4-(4-(4-(3-(2-(4-chlorophenyl)-1-isopropyl-4-methylsulfonyl-5-methyl-1H-pyrrol-3-yl)-5-fluorophenyl)piperazin-1-yl)-phenylaminosulfonyl)-2-trifluoromethylsulfonyl-anilino)-4-phenylthio-butyl)-piperidine-4-carboxylic acid 3-phosphonopropyl ester (Compound D), or a pharmaceutically acceptable salt or solvate thereof; or 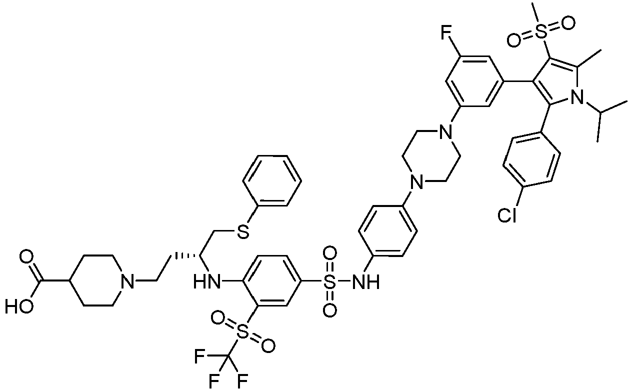
((R)-1-(3-((4-(N-(4-(4-(3-(2-(4-chlorophenyl)-1-isopropyl-5-methyl-4-(methylsulfonyl)-1H-pyrrol-3-yl)-5-fluorophenyl)piperazin-1-yl)phenyl)sulfamoyl)-2-((trifluoromethyl)sulfonyl)phenyl)amino)-4-(phenylthio)butyl)piperidine-4-carboxylic acid (Compound E), or a pharmaceutically acceptable salt or solvate thereof.

39. A kit, comprising:
(a) a first component in a first container, the first component comprising a Bcl-2 inhibitor (preferably a Bcl-2 inhibitor as defined in any one of claims 4 to 6) or a Bcl-2/Bcl-xL inhibitor (preferably a Bcl-2/Bcl-xL inhibitor as defined in Claim 38), and optionally a pharmaceutically acceptable carrier;
(b) a second component in a second container, the second component comprising one or more anticancer reagents (preferably an anticancer reagent as defined in any one of Claims 7 to 12), and optionally a pharmaceutically acceptable carrier; and
(c) an optional specification,
wherein the first container and the second container may be the same or different.
